# EUROPEAN PATENT SPECIFICATION

(11) **EP 1 107 793 B1**
(45) Date of publication and mention of the grant of the patent: **27.10.2004**
(21) Application number: 99939127.9
(22) Date of filing: 10.08.1999
(51) Int. Cl.: A61K 47/48, A61K 38/17, A61P 3/04, A61P 3/06, A61P 3/10

(54) **DEXTRAN-LEPTIN CONJUGATES, PHARMACEUTICAL COMPOSITIONS AND RELATED METHODS**
DEXTRAN-LEPTIN KONJUGATE, PHARMAZEUTISCHE ZUSAMMENTSETZUNGEN UND VERBUNDENE VERFAHREN
CONJUGUES DEXTRANE-LEPTINE, COMPOSITIONS PHARMACEUTIQUES ET METHODES CONNEXES

(30) Priority: 10.08.1998 US 96194 P; 09.08.1999 US 370684
(43) Date of publication of application: 20.06.2001
(73) Proprietor: Amgen Inc., Thousand Oaks, CA 91320-1799 (US)
(72) Inventor: LITZINGER, David, C., Camarillo, CA 93012 (US)
(74) Representative: Brown, John David
(86) International application number: PCT/US1999/018129
(87) International publication number: WO 2000/009165

(56) References cited:
- WO-A-97/06816
- WO-A-97/38014

## Description

### Field

The present invention broadly relates to the field of protein modification and, more specifically, the attachment of dextran moieties to leptin proteins including analogs thereof (the term "protein" as used herein is synonymous with "polypeptide" or "peptide" unless otherwise indicated). In another aspect, the present invention relates to pharmaceutical compositions containing dextran-leptin conjugates. The present invention also provides related compositions and methods of making and using such compositions.

### Background

Although the molecular basis for obesity is largely unknown, the identification of the "OB gene" and its encoded protein ("OB protein" or "leptin") has shed some light on mechanisms the body uses to regulate body fat deposition. See PCT WO 96/05309, entitled "Modulators of Body Weight, Corresponding Nucleic Acids and Proteins, and Diagnostic and Therapeutic Uses Thereof," herein incorporated by reference in its entirety; Zhang *et al*., Nature 372:425-32 (1994); see also, the Correction at Nature 374:479 (1995), both of which are also herein incorporated by reference. The OB protein is active in vivo in both ob/ob mutant mice (mice obese due to a defect in the production of the OB gene product) as well as in normal, wild type mice. The biological activity manifests itself in, among other things, weight loss. See generally, Barinaga, "Obese" Protein Slims Mice," Science 269:475-76 (1995). The OB protein, analogs, derivatives and use thereof as modulators for the control of weight and adiposity of animals, including mammals and humans, has been disclosed in greater detail in WO 96/05309, supra. See also, PCT International Publication Numbers WO 96/40912, WO 97/06816, 97/18833, WO 97/38014, WO 98/08512 and WO 98/28427. The OB protein, or leptin, as it is called herein, causes weight loss in humans. Greenberg et al., "Preliminary safety and efficacy of recombinant methionyl human leptin (rL) administered by SC injection in lean and obese subjects." Poster presented at: 58th Annual Meeting and Scientific Sessions of the American Diabetes Association; June 14, 1998; Chicago, IL.

It is known that the continuous administration of a leptin protein into the systemic circulation, by, for example, osmotic pump or by chemically modifying leptin to have an increased circulation time, reduces dosages necessary for weight loss. E.g., PCT WO 96/40912, entitled "OB Protein Compositions and Methods," herein incorporated by reference. Generally, advantages sought by protein formulation and chemical modification may include, under certain circumstances, increasing the stability and circulation time of the therapeutic protein and decreasing immunogenicity. A review article describing protein modification and fusion proteins is Francis, *Focus on Growth Factors* 3: 4-10 (May 1992) (published by Mediscript, Mountview Court, Friern Barnet Lane, London N20, OLD, UK). Various means for attaching chemical moieties are currently available, see, e.g., Patent Cooperation Treaty ("PCT") International Publication No. WO 96/11953, entitled "N-Terminally Chemically Modified Protein Compositions and Methods," herein incorporated by reference in its entirety. This PCT publication discloses, among other things, the selective attachment of water soluble polymers to the N-terminus of proteins.

Polyethylene glycols are one type of water soluble polymer which may be used for protein modification. See WO 96/11953, supra, which also discloses N-terminally monopegylated granulocyte colony stimulating factor ("G-CSF") and N-terminally monopegylated consensus interferon ("N-terminally monopegylated" denoting that the protein moiety has attached to it a single polyethylene glycol moiety at the N-terminus). Polyethylene glycol moieties may be used with much success for some therapeutic proteins, such as G-CSF and megakaryocyte growth and development factor ("MGDF") (Sheridan & Menchaca, "Overview of the Safety and Biologic Effects of PEG-rHuMGDF in Clinical Trials," in Stem Cells 16(suppl. 2):193-98 (1998)).

Polysaccharide polymers are another type of water soluble polymer which may be used for protein modification. Dextrans are polysaccharide polymers comprised of individual subunits of glucose predominantly linked by α1-6 linkages. The dextran itself is available in many molecular weight ranges, and is readily available in molecular weights from about 1 kiloDalton ("kD") to about 70 kD (the term "about" being used to signify the average molecular weight in a typical commercial preparation of pharmaceutical grade dextran as some dextran molecules may weigh slightly above the stated weight, and some below). Dextran is a suitable water soluble polymer for protein modification. See WO 96/11953, supra, and WO 96/05309, supra. The use of dextran conjugated to therapeutic or diagnostic immunoglobulins also has been reported. European Patent ("EP") Publication No. 0 315 456, entitled "Polysaccharide-Modified Immunoglobulins Having Reduced Immunogenic Potential or Improved Pharmacokinetics," herein incorporated by reference, reports immunoglobulins or fragments thereof, linked to modified low molecular weight polysaccharides, including dextrans.

There is substantial clinical experience in using large amounts of dextran in solution as plasma extenders (volume expanders). See, e.g., Remington's Pharmaceutical Sciences, 18^{th} ed., at 804-05 (Mack Publishing Co.: Easton, PA (1990)), herein incorporated by reference. Solutions containing relatively large molecular weight dextrans, such as dextrans with molecular weights of about 40, 70 and 75 kD, are available and administrated in gram amounts. Dextran-iron solutions are also used in the treatment of anemia.

Large molecular weight dextrans, when administered in gram amounts, are reported to result in kidney vacuoles. E.g., Diomi et al., Annals of Surgery 172:813-24 (1970) (reporting kidney vacuolization in dogs); Maunsbach et al., Laboratory Investigation 11:421-32 (1962) (reporting kidney vacuolization in rats); see also, Engberg, Acta Chir. Scand. 142:172-80 (1976). Polyethylene glycol-protein conjugates, in particular instances, have also been associated with kidney vacuole induction. Bendele et al., Toxicological Sciences 42: 152-57 (1998). Although kidney vacuoles are not currently understood to be clinically relevant, in general, a pharmaceutical composition should be efficacious without causing unwarranted anatomical changes. Thus, large molecular weight dextrans and polyethylene glycol polymers may not be generally applicable for chemical modification of all therapeutic proteins.

Another disadvantage to using large molecular weight dextran in a clinical setting is the possibility of an anaphylactic reaction in the patient. See, e.g., Richter et al., Immunology Today 3:132-38 (1982), herein incorporated by reference, as well as the references cited therein, for a review. It is believed that certain individuals may have in their systemic circulation pre-formed antibodies which bind to these large molecular weight dextrans. Administration of large molecular weight clinical dextran to a small (but unpredictable) subpopulation of individuals with high titers of these pre-formed dextran reactive antibodies ("DRA") of the IgG class results in anaphylactic shock, and possibly death. It is believed that clinical dextrans generate harmful immune complexes, which activate complement and aggregation of leukocytes and platelets occurs. The aggregated material may be sequestered in the lung and release of vasoactive mediators may lead to anaphylactic reactions. Richter et al., supra, at 136.

To overcome this potential risk, dextran solutions containing dextran with a molecular weight of about 1000 D have been studied as hapten inhibitors of the pre-formed DRA. A dextran fragment of six glucose units (molecular weight 990) was found suitable as a monovalent hapten preparation for in-vivo experiments. Richter et al., supra, at 136 et seq. The use of hapten inhibition to reduce anaphylactic reactions has been quite successful for blood expanders. E.g., Ljungstrom, Infusionsther Transfusionsmed 20:206-10 (1993), herein incorporated by reference. In the years 1983 to 1992, the use of a commercial preparation called Promit® is reported to have reduced the incidence of severe dextran induced anaphylactic reactions ("DIAR") dramatically. Fatal reactions were reported at an incidence of one in 2.5 million doses.

EP 0 315 456, as noted above, reports the use of dextrans having a molecular weight of about 6 kD for conjugation to immunoglobulins. It is reported that monoclonal antibodies thought to be useful for therapeutic purposes conjugated with dextrans have reduced immunogenicity while retaining desired immunoreactivity, and may possess desired pharmacokinetic properties. See also, Mikolajczyk et al., Bioconjugate Chem. 7:150-58 (1996) (Fab' component of a Fab'-βlactamase conjugate); Fagnani et al., Nuclear Medicine Comm. 16:362-69 (1995) (Fab' fragments of a murine anti-carcinoembryonic antigen monoclonal antibody); Fagnani et al., Cancer Res. 50:3638-45 (1990) (murine and rabbit immunoglobulins).

It would therefore be desirable to have pharmaceutical compositions containing dextran-leptin conjugates which are non-anaphylactic to individuals with circulating, pre-formed DRA. Moreover, it would be particularly desirable to have dextran-leptin conjugates which exhibit desired characteristics of, as compared to unmodified leptin, extended circulation time, improved efficacy and solubility. The present invention provides dextran-leptin conjugates which have the advantages of chemically modified leptin proteins without the potential risks associated with polyethylene glycol or anaphylaxis-inducing dextrans.

### Summary of the Invention

The present invention stems from the observation that certain relatively low molecular weight dextran-leptin conjugates surprisingly possess the desirable characteristics of improved efficacy and circulation time and possess other desired characteristics such as increased solubility and reduction in injection site reactions as compared to native human leptin. In addition, the potential disadvantage of kidney vacuolization, seen with certain polyethylene glycol-leptin conjugates, was not observed with the subject dextran-leptin conjugates.

The working examples set forth below demonstrate dextran-leptin conjugates which have the following characteristics: (a) improved efficacy over unmodified recombinant methionyl human leptin ("rmetHu-leptin"); (b) an extended plasma circulation time over unmodified rmetHu-leptin; (c) improved aqueous solubility at physiologic pH over unmodified rmetHu-leptin; (d) mild or non-existent injection site reactions; (e) non-immunogenicity of the dextran-leptin conjugate; and (f) no induction of kidney vacuolization.

Therefore, in one aspect, the present invention provides dextran-leptin conjugates comprising at least one low molecular weight dextran moiety attached to at least one leptin moiety wherein the dextran moiety has a molecular weight from 1 kD to 20 kD. Preferably for ease in commercial manufacture of a pharmaceutical product, the dextran moiety has a molecular weight from 1 kD to 10 kD, and more preferably still from 1 kD to 7 kD. A particularly preferred dextran moiety is about 6kD, as exemplified in the Examples below.

In another aspect of the present invention, dextran-leptin conjugates are provided wherein one low molecular weight dextran moiety is attached to one or more leptin moieties. In still another aspect of the invention, dextran-leptin conjugates are provided wherein at least two low molecular weight dextran moieties are attached to one or more leptin moieties. Thus, the present invention includes within its scope a dextran-leptin conjugate mixture containing any combination of at least three predominant species of dextran-leptin conjugates: 1) dextran-leptin conjugate wherein one dextran moiety is attached to one leptin moiety; 2) dextran-leptin conjugate wherein one dextran moiety is attached to two leptin moieties; and 3) dextran-leptin conjugate wherein two dextran moieties are attached to two leptin moieties, the pair of dextran-leptin conjugates being attached to each other. Preferably, the dextran-leptin conjugates of the present invention contain predominantly 1) dextran-leptin conjugate wherein one dextran moiety is attached to two leptin moieties; and 2) dextran-leptin conjugate wherein two dextran moieties are attached to two leptin moieties, the pair of dextran-leptin conjugates being attached to each other.

It is envisioned that the performance characteristics of the dextran-leptin conjugates of the present invention described above may be improved upon increasing the degree of derivatization (i.e., attaching additional dextran moieties to a leptin moiety) without any detrimental effect on anaphylaxis-inducing potential. Thus, dextran-leptin conjugates wherein multiple dextran moieties are attached to the leptin moiety are within the scope of the invention. Such a multi-dextran leptin conjugate would exhibit the same desirable characteristics as described above.

In yet another aspect, the present invention relates to pharmaceutical compositions containing the present dextran-leptin conjugates in a pharmaceutically acceptable carrier. Preferably, the present pharmaceutical compositions contain 1) a dextran-leptin conjugate wherein one dextran moiety is attached to two leptin moieties; and 2) a dextran-leptin conjugate wherein two dextran moieties are attached to two leptin moieties, the pair of dextran-leptin conjugates being attached to each other. A particularly preferred dextran moiety is about 6kD, as exemplified in the dextran-leptin conjugates below.

The present invention also relates to processes for preparing dextran-leptin conjugates as outlined above.

The present invention also relates to medicaments for the treatment of individuals using dextran-leptin conjugates and conjugate mixtures as outlined above.

### BRIEF DESCRIPTION OF THE DRAWINGS

FIGURE 1A is an Endolys-C digestion peptide mapping tracing of unmodified rmetHu-leptin ("Leptin") and dextran-rmetHu-leptin conjugate ("Dextran-Leptin"); FIGURE 1B is an Endoasp-N digestion peptide mapping tracing. The arrow in each tracing indicates the N-terminal peptide of unmodified rmetHu-leptin.
FIGURE 2 presents a 4-20% Tris-Glycine reducing SDS-PAGE gradient gel showing that a significant proportion of the 6 kD dextran leptin conjugate is dimerized. Lane 1 is molecular weight standards (Mark 12, Novex, San Diego, CA); Lane 2 is unmodified rmetHu-leptin as control; Lane 3 is dextran-rmetHu-leptin conjugate mixture; and Lane 4 is dextran-rmetHu-leptin conjugate after ion exchange chromatography.
FIGURE 3 is a graph showing mice data on the amount of weight loss relative to a buffer control versus dose at the end of a seven day, daily dosing study for (a) unmodified rmetHu-leptin ("leptin"); and (b) N-terminal dextran rmetHu-leptin conjugate ("dex-leptin").
FIGURE 4 is a graph showing mice data on the sustained weight-loss effect of the 6 kD dextran-leptin conjugate compared to unmodified rmetHu-leptin in a single dose study. Weight loss is measured as %weight loss relative to a buffer control versus time (days).
FIGURE 5 shows blood circulation times for 6 kD dextran-leptin conjugate compared to unmodified rmetHu-leptin in a single dose rat study. FIGURE 5A shows intravenous injection data; FIGURE 5B shows subcutaneous injection data.
FIGURE 6 is a graph showing mice data on the percentage of weight loss relative to a buffer control versus time at the end of a seven day, daily dosing study for (a) unmodified rmetHu-leptin and (b) 17.5 kD dextran-leptin conjugate.
FIGURE 7 is a graph showing mice data on the percentage of weight loss relative to a buffer control versus time in a single dose study for (a) unmodified rmetHu-leptin and (b) 17.5 kD dextran-leptin conjugate.

### DETAILED DESCRIPTION OF THE INVENTION

The present invention provides dextran-leptin conjugates having the advantages of improved efficacy, longer plasma circulation time and no kidney vacuole formation, among others. Additional advantages of the dextran-leptin conjugates of the present invention include improved solubility and minimal injection site reactions.

### Dextrans

Low molecular weight dextrans used in the practice of this invention include dextrans having a molecular weight from 1 kD to 20 kD, more preferred dextrans having a molecular weight in the range of from 1 kD to 10 kD. More preferred still are dextrans having a molecular weight from 1 kD to 7 kD. A particularly preferred dextran for ease in commercial manufacture of a pharmaceutical for human use is one having a molecular weight of about 6 kD. (The term "about" being used as indicated above in the "Background" section). On average, one mole of 6 kD dextran is composed of 33.3 glucose subunits.

Further preferred, for like reasons, is unbranched dextran. "Native" dextran is produced by Leuconostoc ssp. bacteria. "Clinical" dextran is prepared by the depolymerization of native dextran. From time to time, dextran moieties may be produced such that certain side groups of glucose molecules linked by α1-3 linkages are formed. The formation of such side groups is commonly referred to as "branching" in the art. Although not totally understood at present, large molecular weight dextran moieties with such side groups may be more prone to cause anaphylaxis in humans. While not wishing to be bound by theory, it is believed that there is less potential for branching in low molecular weight dextrans and, in particular, multiple branching, i.e. more than one α1-3 side group per dextran molecule. This is critical because multiple branches are assumed responsible for the aggregation of the preformed dextran reactive antibodies onto dextran thereby causing the formation of large immune complexes. Therefore, preferably, for a human therapeutic product, the dextran moieties for compositions contemplated herein are those with predominantly α1-6 linkages between glucose subunits, and minimizing α1-3 side groups. It has been shown that clinical dextran produced by Leuconostoc mesenteroides strain NRRL B 512 minimizes anaphylactic reactions. Richter et al., supra.

Thus, an important characteristic of the low molecular weight dextrans used in the dextran-leptin conjugates of the present invention is that they are non-anaphylactic to individuals with high titers of circulating, pre-formed DRA.

### Activation of Dextran

The dextran moieties are "activated" so that they may be attached to the leptin protein moiety. Such activation methods are well known to those skilled in the art and include, among others, introducing a chemical moiety on the dextran which can form a bond with a chemical moiety present on the leptin protein. See generally, Larsen, Advanced Drug Delivery Reviews 3:103-54 (1989), herein incorporated by reference. The term "activated" dextran refers to dextran containing multiple reactive groups. The type of "activated" chemical moiety on the dextran will depend on the way one wishes to join the dextran moiety to the protein moiety. For example, one may add an aldehyde group to the dextran so that the dextran moiety may be attached to the leptin moiety under reducing conditions to form an amine bond.

A particularly preferred method of activation is sodium periodate oxidation. The dextran is oxidized to contain multiple aldehyde groups according to a well known procedure. Battersby et al., J. Contr. Rel. 42:143-56 (1996); Fagnani et al. (1990), supra, herein incorporated by reference. A preferred oxidation method is disclosed in the Examples, infra. The molar ratio of periodate to glucose subunit may vary depending on the degree of oxidation desired. Generally, the molar ratio of periodate to glucose subunit may vary from about 0.02:1 to about 3:1, preferably from about 0.1:1 to about 1.5:1. It is envisioned that about 5% to about 50% of the glucose subunits of dextran are oxidized. This percentage represents an average for the total reaction mixture, individual dextran moieties may have a lower or higher percentage. It is particularly preferred that about 10% of the glucose subunits contain aldehyde groups. The term "oxidized" dextran refers to dextran containing multiple aldehyde groups.

### Attachment of Dextran to the Leptin Protein

The dextran moieties should be attached to the leptin moiety with consideration of effects on the functional or antigenic domains of the protein. The method for attachment of the dextran moieties may vary, and there are a number of methods available to those skilled in the art. For example, dextran may be covalently bound to the protein moiety. Covalent binding may take place through amino acid residues via a reactive group, such as a free amine or carboxyl group. Amino acid residues having a suitable amine group may include lysine residues and the N-terminal amino acid residue of the leptin protein moiety. Those amino acids having a free carboxyl group may include aspartic acid residues, glutamic acid residues and the C-terminal amino acid residue of the protein moiety. Sulfhydryl groups may also be used as a reactive group for attachment of the dextran moiety. Alternatively, reactive groups may be introduced into the protein moiety, by, e.g., insertion or site-directed mutagenesis. Preferred for ease in commercial manufacture is attachment at an amino group, such as attachment at the N-terminus of the protein moiety.

Generally, the aldehyde-activated dextran may be attached to the leptin moiety under reducing conditions to form an amine bond. See, Larsen, supra; Fagnani et al. (1990), supra; see also PCT WO 96/11953. The molar ratio of dextran moieties to leptin moieties may range from about 5:1 to about 100:1, preferably about 20:1. Preferably, the pH of the reaction mixture is maintained at or about pH 4.8 in order for the conjugation of the oxidized dextran moiety with the leptin moiety to be N-terminal site specific. The pH should be sufficiently acidic so that the amino terminal amine of the leptin moiety is not yet protonated and, therefore, reactive while the amine groups at other positions on the leptin moiety are protonated rendering them unreactive.

It is envisioned that one of ordinary skill in the art would be able to prepare multiply-reactive leptin moieties by altering reaction parameters such as raising the pH of the reaction mixture, using other existing reactive groups or introducing additional reactive groups to the leptin moiety, as disclosed above. One of ordinary skill would know how to combine any of several known chemical conjugation methods in order to prepare multiply-reactive leptin moieties.

The dextran moiety also may be attached to the leptin moiety by "linker" moieties whether chemical linkers, also known as "cross-linking" reagents, or amino acids of varying lengths. Such chemical linkers are well known in the art and include homobifunctional chemical linkers (i.e. same reactive group at each end of the linker) and heterobifunctional chemical linkers (i.e. different reactive groups at each end of the linker). Considerations such as the kind of reactive groups, the length between reactive ends and other beneficial features of the linker, e.g., an internal metabolizable bond that would allow cleavage of the dextran moiety from the leptin moiety, may impact the choice of chemical linker as is well understood by one of ordinary skill in the art. See, e.g., the Pierce Product Catalog, 1997 (Pierce Chemical Co., Rockford, IL) for a list of cross-linking reagents and references cited therein. The chemical linkers may be attached to the leptin moiety via any of the reactive groups disclosed above in this section. Considerations such as conformation, i.e., flexibility, and size of the peptide may impact the choice of peptide linker as is well understood by one of ordinary skill in the art. E.g., Neve et al., Cytokine 8:365-70 (1996); Hallewell et al., J. Biol. Chem. 264:5260-68 (1989); see generally, Chou & Fasman, Ann. Rev. Biochem. 47:251-76 (1978). Amino acid linker sequences may include but are not limited to:
(a) ala, ala, ala;
(b) ala, ala, ala, ala;
(c) ala, ala, ala, ala, ala;
(d) gly, gly;
(e) gly, gly, gly;
(f) gly, gly, gly, gly, gly;
(g) gly, gly, gly, gly, gly, gly, gly;
(h) gly, pro, gly;
(i) gly, gly, pro, gly, gly; and
(j) any combination of subparts (a) through (i).
The amino acid linker sequences may be attached either at the N-terminus or C-terminus of the leptin moiety by expression of the leptin moiety as a fusion protein.

### Dextran-Leptin Conjugates

Composition of Dextran-Leptin Conjugates. The dextran-leptin conjugates of the present invention may be characterized using well-known methods in the art such as SDS-PAGE analysis, mass spectrometry, RP-HPLC peptide mapping and N-terminal sequence analysis. It has been found that, in general, at least three species of N-terminal dextran-leptin conjugates exist in the reaction mixture after dextran is attached to the leptin moiety as described above. The three species are: 1) one dextran moiety attached to one leptin moiety; 2) one dextran moiety attached to two leptin moieties; and 3) two dextran moieties attached to two leptin moieties. It is believed that the two dextran-two leptin species is formed by a pair of dextran-leptin conjugates being attached to each other. The last two species typically make up approximately 65-85% of the reaction mixture, with the first species typically accounting for 5-10% of the reaction mixture. The remaining minor species appear as unreacted leptin and higher molecular weight entities in SDS-PAGE analysis. These may be removed from the mixture using known methods. After purification, the two dimer species typically make up approximately 70-90% of the dextran-leptin conjugate.

It is envisioned that, if one wished to reduce the propensity of the dextran-leptin conjugate to form a dimer, one of ordinary skill in the art may prepare a leptin analog, as discussed infra, that has a reduced tendency towards dimer formation.

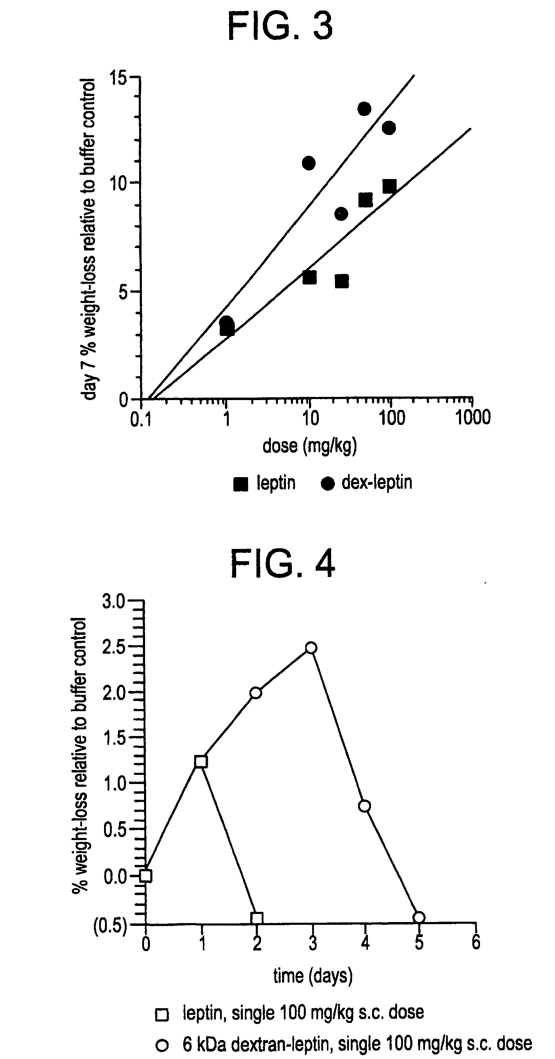

Leptin Proteins. The type of leptin used for the present dextran-leptin conjugates may be selected from those described in PCT International Publication Number WO 96/05309, as cited above and herein incorporated by reference in its entirety. Figure 3 of that publication (as cited therein SEQ ID NO: 4) depicts the full deduced amino acid sequence derived for human leptin (referred to as the human "OB" protein). The amino acids are numbered from 1 to 167. A signal sequence cleavage site is located after amino acid 21 (Ala) so that the mature protein extends from amino acid 22 (Val) to amino acid 167 (Cys). For the present disclosure, a different numbering is used herein, where the amino acid position 1 is the valine residue which is at the beginning of the mature protein. The amino acid sequence for mature, recombinant methionyl human leptin is presented herein as SEQ ID NO: 1, where the first amino acid of the mature protein is valine (at position 1) and a methionyl residue is located at position -1 (not included in the sequence below). 
V P I Q K V Q D D T K T L I K T I V
T R I N D I S H T Q S V S S K Q K V T G
L D F I P G L H P I L T L S K M D Q T L
A V Y Q Q I L T S M P S R N V I Q I S N
D L E N L R D L L H V L A F S K S C H L
P W A S G L E T L D S L G G V L E A S G
Y S T E V V A L S R L Q G S L Q D M L W
Q L D L S P G C
 However, as with any of the present leptin moieties, the methionyl residue at position -1 may be absent.

Alternatively, one may use a natural variant of human leptin, which has 145 amino acids and, as compared to rmetHu-leptin of SEQ ID NO: 1, has a glutamine absent at position 28.

Generally, the leptin moiety for human pharmaceutical use herein will be capable of therapeutic use in humans (see also, animal leptins, below). Thus, one may empirically test activity to determine which leptin moieties may be used. As set forth in WO 96/05309, leptin protein in its native form, or fragments (such as enzyme cleavage products) or other truncated forms and analogs may all retain biological activity. Any of such forms may be used as a leptin moiety for the present dextran-leptin conjugates, although such altered forms should be tested to determine desired characteristics. See also, PCT International Publication Numbers WO 96/40912, WO 97/06816, 97/18833, WO 97/38014, WO 98/08512 and WO 98/28427, herein incorporated by reference in their entireties.

One may prepare an analog of recombinant human leptin by altering amino acid residues in the recombinant human sequence, such as substituting the amino acids which diverge from the murine sequence. Murine leptin is substantially homologous to human leptin, particularly as a mature protein and, further, particularly at the N-terminus. Because the recombinant human protein has biological activity in mice, such an analog would likely be active in humans. For example, in the amino acid sequence of native human leptin as presented in SEQ ID NO: 1, one may substitute with another amino acid one or more of the amino acids at positions 32, 35, 50, 64, 68, 71, 74, 77, 89, 97, 100, 101, 105, 106, 107, 108, 111, 118, 136, 138, 142 and 145. One may select the amino acid at the corresponding position of the murine protein (see Zhang et al., 1994, supra) or another amino acid.

One may further prepare "consensus" molecules based on the rat OB protein sequence. Murakami et al., Biochem. Biophys. Res. Comm. 209: 944-52 (1995) herein incorporated by reference. Rat OB protein differs from human OB protein at the following positions (using the numbering of SEQ ID NO: 1): 4, 32, 33, 35, 50, 68, 71, 74, 77, 78, 89, 97, 100, 101, 102, 105, 106, 107, 108, 111, 118, 136, 138 and 145. One may substitute with another amino acid one or more of the amino acids at these divergent positions. The positions underlined are those in which the murine OB protein as well as the rat OB protein are divergent from the human OB protein and, thus, are particularly suitable for alteration. At one or more of the positions, one may substitute an amino acid from the corresponding rat OB protein, or another amino acid.

The positions from both rat and murine OB protein which diverge from the mature human OB protein are: 4, 32, 33, 35, 50, 64, 68, 71, 74, 77, 78, 89, 97, 100, 101, 102, 105, 106, 107, 108, 111, 118, 136, 138, 142 and 145. An OB protein according to SEQ ID NO: 1 having one or more of the above amino acids replaced with another amino acid, such as the amino acid found in the corresponding rat or murine sequence, may also be effective.

In addition, the amino acids found in rhesus monkey OB protein which diverge from the mature human OB protein are (with identities noted in parentheses in one letter amino acid abbreviation): 8 (S), 35 (R), 48 (V), 53 (Q), 60 (I), 66 (I), 67 (N), 68 (L), 89 (L), 100 (L), 108 (E), 112 (D) and 118 (L). Since the recombinant human OB protein is active in cynomolgus monkeys, a human OB protein according to SEQ ID NO: 1 having one or more of the rhesus monkey divergent amino acids replaced with another amino acid, such as the amino acids in parentheses, may be effective. It should be noted that certain rhesus divergent amino acids are also those found in the above murine and rat species (positions 35, 68, 89, 100, 108 and 118). Thus, one may prepare a murine/rat/rhesus/human consensus molecule (using the numbering of SEQ ID NO: 1) having one or more of the amino acids replaced by another amino acid at positions: 4, 8, 32, 33, 35, 48, 50, 53, 60, 64, 66, 67, 68, 71, 74, 77, 78, 89, 97, 100, 102, 105, 106, 107, 108, 111, 112, 118, 136, 138, 142 and 145. The positions underlined are those in which all three species are divergent from human OB protein. A particularly preferred human leptin analog is one wherein the amino acids at position 100 (Trp) or 138 (Trp), and more preferably, both positions are substituted with another amino acid, preferably Gln.

Other analogs may be prepared by deleting a part of the protein amino acid sequence. For example, the mature protein lacks a leader sequence (-22 to -1). One may prepare the following truncated forms of human OB protein molecules (using the numbering of SEQ ID NO: 1):
(i) amino acids 98-146;
(ii) amino acids 1-99 and (connected to) 112-146;
(iii) amino acids 1-99 and (connected to) 112-146 having one or more of amino acids 100-111 sequentially placed between amino acids 99 and 112.

In addition, the truncated forms may also have altered one or more of the amino acids which are divergent (in the murine, rat or rhesus OB protein) from human OB protein. Furthermore, any alterations may be in the form of altered amino acids, such as peptidomimetics or D-amino acids.

Also included are those proteins as set forth above with amino acid substitutions which are "conservative" according to acidity, charge, hydrophobicity, polarity, size or any other characteristic known to those skilled in the art. These are set forth in Table 1, below. See generally, Creighton, Proteins, passim (W.H. Freeman and Company, N.Y., 1984); Ford et al., Protein Expression and Purification 2:95-107 (1991), which are herein incorporated by reference.

**Table 1**

| Conservative Amino Acid Substitutions | |
|---|---|
| Basic: | arginine lysine histidine |
| Acidic: | glutamic acid aspartic acid |
| Polar: | glutamine asparagine |
| Hydrophobic: | leucine isoleucine valine |
| Aromatic: | phenylalanine tryptophan tyrosine |
| Small: | glycine alanine serine threonine methionine |

Therefore, the present dextran-leptin conjugates may be selected from among (according to the amino acid sequence as presented in SEQ ID NO: 1 herein):
(a) the amino acid sequence of SEQ ID NO: 1, optionally lacking a glutaminyl residue at position 28, and further optionally having a methionyl residue at the N-terminus;
(b) an amino acid sequence of subpart (a) having a different amino acid substituted in one or more of the following positions: 4, 8, 32, 33, 35, 48, 50, 53, 60, 64, 66, 67, 68, 71, 74, 77, 78, 89, 97, 100, 102, 105, 106, 107, 108, 111, 112, 118, 136, 138, 142 and 145;
(c) an amino acid sequence of subpart (b) wherein the amino acids at positions 100 and 138 are substituted with Gln;
(d) a truncated leptin protein analog selected from among:
   (i) amino acids 98-146
   (ii) amino acids 1-99 and 112-146
   (iii) amino acids 1-99 and 112-146 having one or more of amino acids 100-111 sequentially placed between amino acids 99 and 112; and,
   (iv) the truncated leptin analog of subpart (i) having one or more of amino acids 100, 102, 105, 106, 107, 108, 111, 112, 118, 136, 138, 142 and 145 substituted with another amino acid;
   (v) the truncated leptin analog of subpart (iii) having one or more of amino acids 4, 8, 32, 33, 35, 48, 50, 53, 60, 64, 66, 67, 68, 71, 74, 77, 78, 89, 97, 112, 118, 136, 138, 142 and 145 replaced with another amino acid;
   (vi) the truncated leptin analog of subpart (iv) having one or more of amino acids 4, 8, 32, 33, 35, 48, 50, 53, 60, 64, 66, 67, 68, 71, 74, 77, 78, 89, 97, 100, 102, 105, 106, 107, 108, 111, 112, 118, 136, 138, 142 and 145 replaced with another amino acid; and
   (vii) the truncated leptin analog of any of subparts (i)-(vi) having an N-terminal methionyl residue;
(e) a leptin protein of any of subparts (a)-(d) having one or more conserved amino acid substitutions.

Leptin proteins, analogs and related molecules are also reported in the following publications; however, no representation is made with regard to the activity of any composition reported:
U.S. Patent Nos. 5,521,283; 5,525,705; 5,532,336; 5,552,522; 5,552,523; 5,552,524; 5,554,727; 5,559,208; 5,563,243; 5,563,244; 5,563,245; 5,567,678; 5,567,803; 5,569,743; 5,569,744; 5,574,133; 5,580,954; 5,594,101; 5,594,104; 5,605,886; 5,614,379; 5,691,309; 5,719,266 (Eli Lilly and Company);
PCT WO96/23513; WO96/23514; WO96/23515; WO96/23516;
WO96/23517; WO96/23518; WO96/23519; WO96/23520;
WO96/23815; WO96/27385; WO96/34111; WO96/37517;
WO97/00886; EP 725078; EP 725079; EP 744408; EP 745610; EP 835879 (Eli Lilly and Company);
PCT WO96/22308 (Zymogenetics);
PCT WO96/31526 (Amylin Pharmaceuticals, Inc.);
PCT WO96/34885; WO97/46585 (Smithkline Beecham PLC);
PCT WO96/35787 (Chiron Corporation);
PCT WO97/16550 (Bristol-Myers Squibb);
PCT WO97/20933 (Schering Corporation)
EP 736599 (Takeda);
EP 741187 (F. Hoffman LaRoche).

To the extent these references provide for useful leptin proteins or analogs, or associated compositions or methods, such compositions and/or methods may be used in conjunction with the present dextran-leptin conjugates, such as for co-administration (together or separately, in a selected dosage schedule).

Specifically contemplated are the following dextran-leptin conjugates: recombinant human leptin ("rHu-leptin"), the amino acid sequence of SEQ ID NO: 1, optionally lacking a glutaminyl residue at position 28, and further optionally having an N-terminal methionyl residue, attached to (a) a dextran moiety solely at the N-terminus; or (b) a dextran moiety at the N-terminus in combination with additional dextran moieties at positions other than the N-terminus of the leptin protein. In particular, the dextran moieties of the present dextran-leptin conjugates may have a molecular weight in the range of from 1 kD to 20 kD, more particularly, from 1 kD to 10 kD, even more particularly, from 1 kD to 7 kD and, most particularly, having a molecular weight of about 6 kD. Also specifically contemplated is a dextran-leptin conjugate made up of a 6 kD dextran moiety attached at the N-terminus to rmetHu-leptin. The dextran moieties of the present dextran-leptin conjugates are preferably made from a strain of bacteria that minimizes α1-3 branching.

Also specifically contemplated are dextran-leptin conjugate mixtures comprising the following in any combination:
(a) a dextran-leptin conjugate wherein one dextran moiety is attached to one leptin moiety;
(b) a dextran-leptin conjugate wherein one dextran moiety is attached to two leptin moieties; and
(c) a dextran-leptin conjugate wherein two dextran moieties are attached to two leptin moieties, said pair of dextran-leptin conjugates being attached to each other;
wherein the dextran moiety is attached at the N-terminus to the leptin moiety having the amino acid sequence of SEQ ID NO: 1, optionally lacking a glutaminyl residue at position 28, and further optionally having a methionyl residue at the N-terminus.

### Animal Leptins

In addition to the above human therapeutic leptin, certain animal leptins are also available for animal therapeutic use. Canine leptin is disclosed in WO 97/32022. Other animal species are disclosed in the following publications: WO 96/36644, EP 743321 (porcine and bovine); WO 98/04288 (bovine); WO 98/04690 (porcine).

### Pharmaceutical Compositions

In yet another aspect of the present invention, provided are pharmaceutical compositions of the present dextran-leptin conjugates and medicaments using such pharmaceutical compositions for therapeutic uses. Such pharmaceutical compositions may be for administration by bolus injection or by infusion (e.g., intravenous or subcutaneous), or for oral, pulmonary, nasal, transdermal or other forms of administration. In general, comprehended by the invention are pharmaceutical compositions comprising effective amounts of dextran-leptin conjugates of the invention together with pharmaceutically acceptable diluents, preservatives, solubilizers, emulsifiers, adjuvants and/or carriers. Such pharmaceutical compositions include diluents of various buffer content (e.g., Tris-HCl, acetate, phosphate), pH and ionic strength; additives such as detergents and solubilizing agents (e.g., Tween 80, Polysorbate 80), anti-oxidants (e.g., ascorbic acid, sodium metabisulfite), preservatives (e.g., Thimersol, benzyl alcohol) and bulking substances (e.g., lactose, mannitol); incorporation of the material into particulate preparations of polymeric compounds such as polylactic acid, polyglycolic acid, etc. or into liposomes. See, e.g., PCT WO 96/29989. Hylauronic acid may also be used, and this may have the effect of increasing sustained duration in the circulation. Such compositions may influence the physical state, stability, rate of in vivo release, and rate of in vivo clearance of the present dextran-leptin conjugates. See, e.g., Remington's Pharmaceutical Sciences, 18th Ed. (1990, Mack Publishing Co., Easton, PA 18042) pages 1435-1712. The compositions may be prepared in liquid form, or may be in dried powder, such as lyophilized form. Implantable sustained release formulations are also contemplated, as are transdermal formulations.

Contemplated are oral formulations, as described in PCT WO 95/21629. This PCT publication describes oral delivery of chemically modified proteins, including proteins modified by dextran moieties. The compositions and methods disclosed therein are applicable here to prepare oral delivery formulations of the present dextran-leptin conjugates.

Pulmonary delivery of the present dextran-leptin conjugates is also contemplated, and compositions and methods disclosed in PCT WO 94/20069 are useful for the preparation and use of the present dextran-leptin conjugates. WO 94/20069 discloses the pulmonary delivery of chemically-modified G-CSF,

The dextran-leptin conjugates of the present invention should most advantageously be prepared in particulate form with an average particle size of less than 10 microns, most preferably 0.5 to 5 microns, for most effective delivery to the distal lung.

Nasal delivery of the present dextran-leptin conjugates is also contemplated. Nasal delivery allows the passage of the protein to the blood stream directly after administering the therapeutic product to the nose, without the necessity for deposition of the product in the lung. Formulations for nasal delivery include those with dextran or dextrin, such as cyclodextrin. Delivery via transport across other mucus membranes is also contemplated.

### Dosages

One skilled in the art will be able to ascertain effective dosages by administration and observing the desired therapeutic effect. Preferably, the formulation of the conjugate will be such that between about 0.01 µg leptin moiety/kg body weight/day and 10 mg leptin moiety/kg body weight/day will yield the desired therapeutic effect. The effective dosages may be determined using diagnostic tools over time. For example, a diagnostic for measuring the amount of leptin in the blood (or plasma or serum) may first be used to determine endogenous levels of leptin protein. Such diagnostic tool may be in the form of an antibody assay, such as an antibody sandwich assay. The amount of endogenous leptin protein is quantified initially, and a baseline is determined. The therapeutic dosages are determined as the quantification of endogenous and exogenous leptin protein moiety (that is, protein, analog or derivative found within the body, either self-produced or administered) is continued over the course of therapy. The dosages may therefore vary over the course of therapy, with, for example, a relatively high dosage being used initially, until therapeutic benefit is seen, and lower dosages used to maintain the therapeutic benefits.

### Uses

Therapeutic. Therapeutic uses include weight modulation, the treatment or prevention of diabetes, blood lipid reduction (and treatment of related conditions), increasing lean body mass and increasing insulin sensitivity. In addition, the present compositions may be used for manufacture of one or more medicaments for treatment or amelioration of the above conditions.

Weight Modulation. The present compositions may be used for weight reduction. Viewed another way, the present compositions may be used for maintenance of a desired weight or level of adiposity. As has been demonstrated in murine models (see infra), administration of the present dextran-leptin conjugates results in weight loss. The body mass lost is primarily of adipose tissue, or fat. Such weight loss can be associated with the treatment of concomitant conditions, such as those below, and therefore constitute a therapeutic application. In addition, cosmetic uses are provided herein if weight modulation is solely for improvement in appearance.

Treatment of Diabetes. The present compositions may be used in the prevention or treatment of Type II diabetes. As Type II diabetes can be correlated with obesity, use of the present invention to reduce weight (or maintain a desired weight, or reduce or maintain an adiposity level) can also alleviate or prevent the development of diabetes. Moreover, even in the absence of dosages sufficient to result in weight loss, the present compositions may be used to prevent or ameliorate diabetes.

Blood Lipid Modulation. The present compositions may be used in the modulation of blood lipid levels. Hyperlipidemia (also called lipemia; dyslipidemia) is the presence of an abnormally large amount of lipids in the circulating blood. Ideally, in situations where solely reduction in blood lipid levels is desired, or where maintenance of blood lipid levels is desired, the dosage will be insufficient to result in weight loss. Thus, during an initial course of therapy of an obese patient, dosages may be administered whereby weight loss and concomitant blood lipid level lowering is achieved. Once sufficient weight loss is achieved, a dosage sufficient to prevent re-gaining weight, yet sufficient to maintain desired blood lipid levels, or other conditions as set forth herein, for example, may be administered. These dosages can be determined empirically, as the effects of leptin protein are reversible. E.g., Campfield et al., Science 269: 546-549 (1995) at 547. Thus, if a dosage resulting in weight loss is observed when weight loss is not desired, one would administer a lower dose in order to achieve the desired blood lipid levels, yet maintain the desired weight. See, e.g., PCT Publication WO 97/06816 herein incorporated by reference.

Increasing Lean Mass or Insulin Sensitivity. Ideally, in situations where solely an increase in lean body mass is desired, the dosage will be insufficient to result in weight loss. Thus, during an initial course of therapy of an obese person, dosages may be administered whereby weight loss and concomitant fat tissue decrease/lean mass increase is achieved. Once sufficient weight loss is achieved, a dosage sufficient to prevent regaining weight, yet sufficient to maintain desired lean mass increase (or prevention of lean mass depletion) may be administered. For increasing an individual's sensitivity to insulin, similar dosage considerations may be taken into account. Lean mass increase without weight loss may be achieved sufficient to decrease the amount of insulin (or, potentially, amylin, amylin antagonists or agonists, or thiazolidinediones, or other potential diabetes treating drugs) an individual would be administered for the treatment of diabetes. For increasing overall strength, there may be similar dosage considerations. Lean mass increase with concomitant increase in overall strength may be achieved with doses insufficient to result in weight loss. Other benefits, such as an increase in red blood cells (and oxygenation in the blood) and a decrease in bone resorption or osteoporosis may also be achieved in the absence of weight loss. See, e.g., PCT Publication No. WO 97/18833.

Combination Therapies. The present compositions may be used in conjunction with other therapies, such as altered diet and exercise. Other medicaments, such as those useful for the treatment of diabetes (e.g., insulin and possibly amylin, antagonists or agonists thereof, thiazolidinediones (see, e.g., PCT Publication No. WO 98/08512), or other potential diabetes treating drugs), cholesterol and blood pressure lowering medicaments (such as those which reduce blood lipid levels or other cardiovascular medicaments), activity increasing medicaments (e.g., amphetamines), diuretics (for liquid elimination), and appetite suppressants (such as agents which act on neuropeptide Y receptors or serotonin reuptake inhibitors). Such administration may be simultaneous or may be in seriatim. In addition, the present methods may be used in conjunction with surgical procedures, such as cosmetic surgeries designed to alter the overall appearance of a body (e.g., liposuction or laser surgeries designed to reduce body mass, or implant surgeries designed to increase the appearance of body mass). The health benefits of cardiac surgeries, such as bypass surgeries or other surgeries designed to relieve a deleterious condition caused by blockage of blood vessels by fatty deposits, such as arterial plaque, may be increased with concomitant use of the present compositions and methods. Methods to eliminate gall stones, such as ultrasonic or laser methods, may also be used either prior to, during or after a course of the present therapeutic methods. Furthermore, the present methods may be used as an adjunct to surgeries or therapies for broken bones, damaged muscle, or other therapies which would be improved by an increase in lean tissue mass.

### Methods of Leptin Production

The leptin moieties used herein may be made in prokaryotic or in eukaryotic cells, although, for the leptin moieties used in the working examples below, bacteria is preferred for ease in commercial manufacture. One may further use leptin made in human cells, such as that made by controlling a native or introduced regulatory element which affects the regulation of an endogenous gene encoding the desired protein. Recombinant expression of leptin moieties useful in the dextran-leptin conjugates of the present invention has been described, for example, in WO 96/40912, including all vector and host strain deposits cited therein.

### Purification of Conjugates

Selected dextran-leptin conjugates of the present invention may be isolated from dextran-leptin conjugate mixtures using well known methods in the art for purifying proteins. A one step purification procedure can be easily achieved by performing cation exchange chromatography. See, e.g., Ralph et al., Biochemistry 34:4889-97 (1995). Another known purification method useful in purifying the dextran-leptin conjugates of the present invention involves hydrophobic interaction chromatography. See, e.g., Pharmacia Biotech's HiTrap HIC Test Kit Instructions, 71-7147-00, ed. AF, at 1-19 (1993; Uppsala, Sweden).

The first set of examples below demonstrate with 6 kD dextran-rmetHu-leptin conjugate (1) activity; (2) increased circulation time in vivo as compared to native rmetHu-leptin; (3) increased solubility under physiologic conditions (as compared to native rmetHu-leptin); (4) lack of injection site reactions; (5) minimal immunogenic response in primates; and (6) lack of kidney vacuolization. The second set of examples below demonstrate some of the same performance characteristics as above with 17.5 kD dextran-rmetHu-leptin conjugate.

### EXAMPLES

Animals. Test animals used in the following experiments were housed five to a cage for mice and two to a cage for rats; all animals were fed ad libitum. A twelve hour light/dark cycle was maintained throughout the experiments. The animals were handled and cared for in accordance with accepted practices for care of laboratory animals.

Administration of Dextran-Leptin. Administration of either test composition or placebo was by subcutaneous ("s.c.") injection into the dorsal scruff of the neck of the animals or intravenously through an indwelling catheter into the jugular vein ("i.v."). All dosings of dextran-leptin conjugate were calculated by determining the leptin protein concentration of the conjugate.

Leptin Protein. Recombinant methionyl human leptin (rmetHu-leptin) was used for the present experiments.

### Example 1

This example shows the preparation of a 6 kD dextran-leptin conjugate of the present invention.

Activation of Dextran. Six kD dextran (peak mol. wt. 6 kD with polydispersity of approximately 1.77 kD) was purchased from Fluka Chemical Corp. (Ronkonkoma, NY). The dextran was activated to add aldehyde groups. Briefly, 0.35 M sodium periodate was added slowly to 10 mM sodium tetraborate on ice, and the pH was adjusted to pH 3.0. One hundred twenty grams of 6kD dextran was added to 400 mL of the above solution, and the mixture was stirred for 24 hours at room temperature. The molar ratio of periodate to glucose subunit was 0.26:1. While continuing to stir, 73.6 mL of ethylene glycol was added and the mixture was stirred for an additional two hours. The oxidized dextran was extensively dialyzed against distilled, deionized H2O.

Both before and after activation, the dextran was tested using gel permeation chromatography (GPC) to confirm the stability of the dextran moiety. After activation, the number of aldehydes on the activated dextran moieties were quantitated using methods well known to one of skill in the art. Zhao & Heindel, Pharm. Res. 8:400-02 (1991). Other dextrans were assayed and activated in a similar fashion.

Attachment of Dextran to the Leptin Protein. Briefly, rmetHu-leptin was made into a solution at a concentration of about 1 mg/mL in 40 mM sodium acetate buffer, pH 4.8, which was mixed with a 10 mg/mL oxidized dextran, 40 mM sodium acetate solution, pH 4.8. The final molar ratio of dextran moieties to leptin moieties was 20:1. The mixture was stirred on a rotating platform for 1 hour at room temperature. A 1 mg/mL solution of NaBH3CN in 40 mM sodium acetate, pH 4.8, was added at a molar ratio of NaBH3CN to dextran of 10:1. The mixture was stirred at room temperature for two hours and then transferred to a cold room (4°C) to continue stirring. The dextran-leptin conjugate was treated with 10 mg/mL NaBH4 in 40 mM sodium acetate, pH 4.8, at a molar ratio of NaBH4 to oxidized glucose units of 2.5:1 (10% or 3.3 mol of oxidized glucose units/mol 6 kD dextran). The NaBH4 solution was added dropwise to the mixture while on ice being stirred.

Characterization. Determination of dextran substitution of conjugates was performed. The purity and approximate molecular weight of the dextran-leptin conjugates were determined by SDS-PAGE analysis.

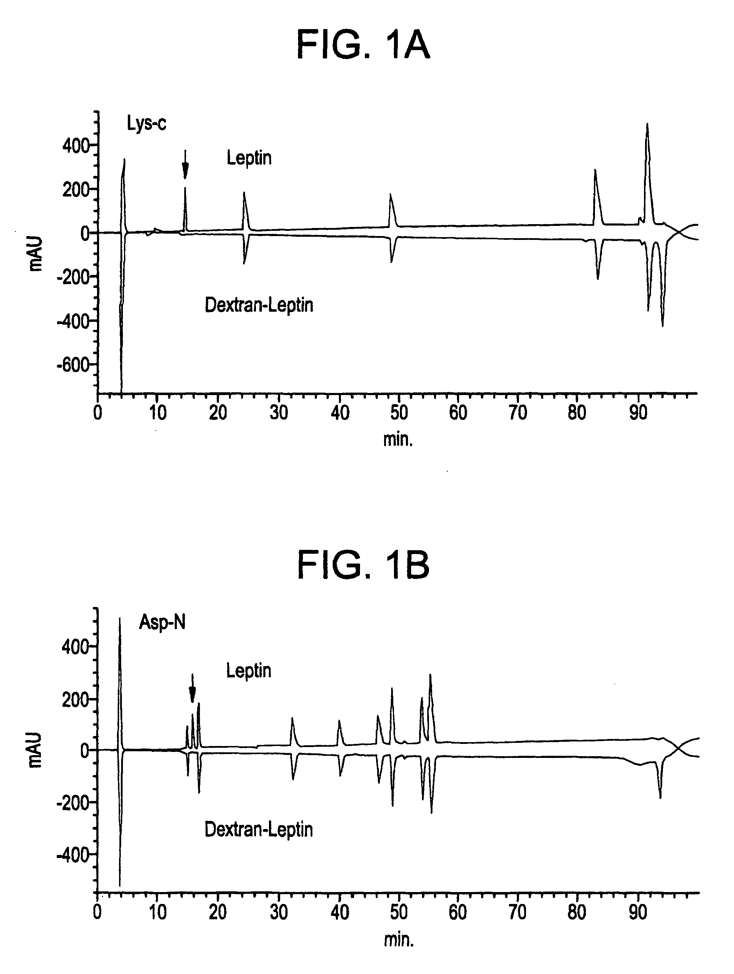

It was determined that the binding of the activated aldehyde groups of the oxidized dextran moieties were attached at the N-terminus of the leptin moieties. Briefly, dextran-rmetHu-leptin conjugate and unmodified rmetHu-leptin were cut by endoproteases at lysine residues (EndoLys-C) or aspartic acid residues (EndoAsp-N), respectively, in two separate experiments. Samples were digested at an enzyme to substrate ratio of 1:50 and 1:75, respectively, at 25°C for 8 and 7 hours, respectively. The resulting peptide fragments were mapped by RP-HPLC using a YMC C-8 column (2.1 mm id.) and eluted using a gradient of two solvents, Solvent A) 0.1% TFA in HPLC grade water and Solvent B) 0.1% TFA in 90% HPLC grade acetonitrile, at the following concentrations: 5 min. 100% A; 80 min. 90% A, 10% B; 5 min. 50% A, 50% B; 10 min. 10% A, 90% B. The column was maintained at 26°C and eluted at 0.2 mL/min. Ralph et al., supra. As compared to unmodified rmetHu-leptin, peptide mapping of the dextran-rmetHu-leptin conjugate with Endolys-C digestion (Fig. 1A) showed that a single peak disappeared at approximately 14 minutes. Similarly, peptide mapping with Endoasp-N digestion (Fig. 1B) showed that a single peak disappeared at approximately 16 minutes. These peptides from the unmodified rmetHu-leptin were N-terminally sequenced for identification and confirmed to be the N-terminal peptides generated by the protease digestions. Thus, the attachment of the dextran moiety to the leptin moiety was selective for the N-terminal peptides. Furthermore, N-terminal sequencing showed approximately 98.6% blocked N-terminus, indicating that the dextran was conjugated to the N-terminus of rmetHu-leptin.

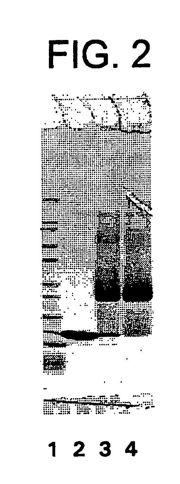

Furthermore, it was determined by SDS-PAGE analysis that the propensity of the reaction was driven towards dimer formation, i.e., the attachment of one dextran moiety to two leptin moieties or two dextran moieties to two leptin moieties. As seen in Figure 2, a significant proportion of the dextran-leptin conjugate presents itself in dimer formation (Lane 3 is dextran-rmetHu-leptin conjugate mixture). It has been determined by Delayed Extraction MALDI-TOF mass spectrometry, using sinapinic acid as matrix, that the main band of Lane 3 consists of both dimer species (i.e., one dextran moiety-two leptin moieties, two dextran moieties-two leptin moieties). Unreacted rmetHu-leptin and some of the higher molecular weight species formed in minor amounts were separated from the majority of dimer conjugates and the minority of monomer conjugates through ion exchange chromatography (Lane 4 shows dextran-rmetHu-leptin conjugate after ion exchange chromatography).

### Example 2

This example demonstrates the improved efficacy of the 6 kD dextran-leptin conjugates of the present invention over unmodified rmetHu-leptin. The dextran-leptin conjugate of Example 1 was tested for efficacy in inducing weight loss in normal mice.

Normal female C57BL/6 mice (Charles River Laboratories, Wilmington, MA), age 8-10 weeks and weighing approximately 20 grams, were injected daily for seven days with either the subject dextran-leptin conjugate (as described in Example 1 above), unmodified leptin, or placebo (phosphate buffered saline, "PBS"). Doses of 1 mg/kg, 10 mg/kg, 25 mg/kg, 50 mg/kg and 100 mg/kg were tested for both the dextran-leptin conjugate and unmodified leptin. Weights were monitored throughout the study.

The weight change at the end of the study (day 7) for the dextran-leptin conjugate-treated group was compared against the unmodified leptin-treated group. The weight loss was calculated as the amount of weight loss relative to a buffer control ((wt. buffer group - wt. sample group)/wt. buffer group x 100). The efficacy of the dextran-leptin conjugate was significantly greater than that for the unmodified leptin at the 10 mg/kg, 25 mg/kg, 50 mg/kg and 100 mg/kg doses. Moreover, a dose-response was observed for the dextran-leptin conjugate. Percent weight loss at the end of the study relative to the PBS buffer control was plotted against dose in mg/kg for both the dextran-leptin conjugate and the unmodified leptin. As shown in Figure 3, by fitting the data to simple logarithmic curves and assuming an ED₅₀ (dose required to achieve 50% maximal weight loss) to give 7% weight loss, one can approximate the ED₅₀ values for the dextran-leptin conjugate and unmodified leptin to be 4 mg/kg and 20 mg/kg, respectively. Thus, the data demonstrates that the dextran-leptin conjugate of the present invention is roughly 5-fold more efficacious than unmodified leptin.

### Example 3

This example demonstrates the sustained weight-loss effect of the 6 kD dextran-leptin conjugates of the present invention. Normal female C57BL/6 mice, age 8-10 weeks and weighing approximately 20 grams, were injected s.c. only on day 0 at a dose of 100 mg/kg with either dextran-leptin conjugate or unmodified leptin. As seen in Figure 4, the weight loss effect of the dextran-leptin conjugate lasted for 4-5 days with a two-fold increase in peak weight loss, whereas the weight loss effect of the unmodified leptin lasted only 1-2 days.

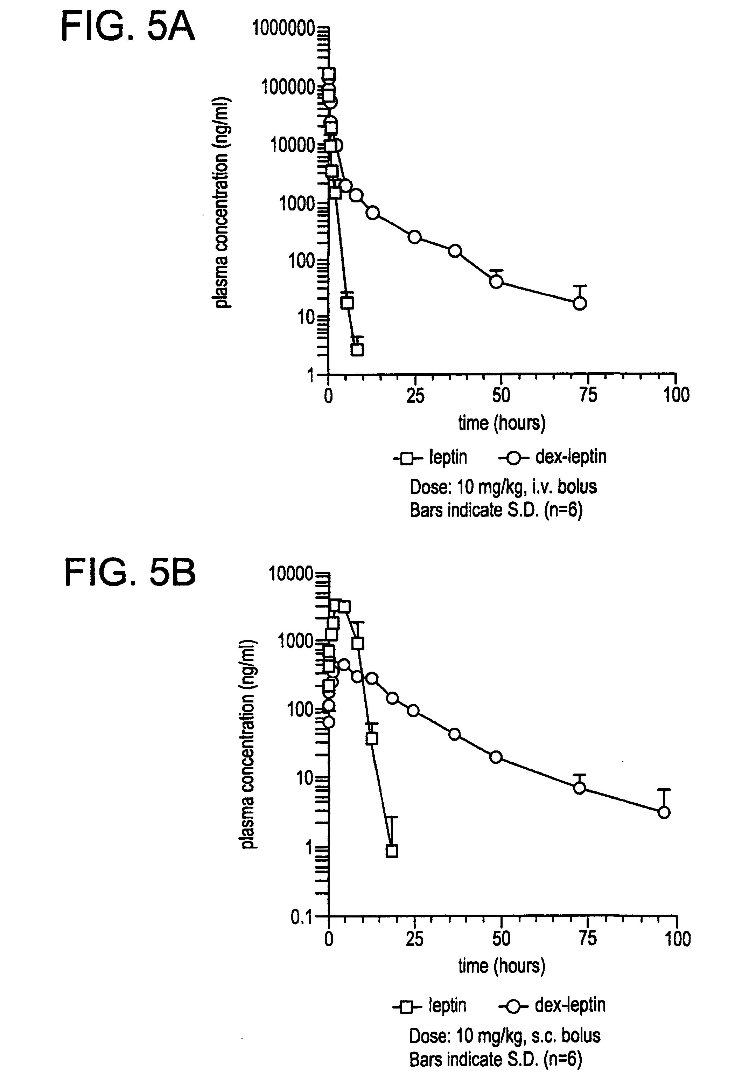

Pharmacokinetic studies in rats showed that the dextran-leptin conjugate persisted in the blood substantially longer than the unmodified leptin. Catheterized male Wistar Kyoto rats weighing 200-250 grams were injected with a single dose at 10 mg/kg of the subject dextran-leptin conjugate (as described in Example 1 above) or unmodified leptin, either s.c. or intravenously ("i.v."). At fixed time intervals post-injection, blood samples were withdrawn via the indwelling catheter. As seen in Figure 5A, the dextran-leptin conjugate injected i.v. persisted in the blood through 72 hours post-injection, whereas the unmodified leptin was completely cleared by about 8-12 hours post-injection. Although both molecules exhibited biphasic clearance, both the initial phase half-life and, particularly, the terminal phase half-life for the dextran-leptin conjugate were significantly increased, indicating that the conjugated dextran slows the clearance of the leptin protein from the bloodstream. Thus, plasma circulation time in vivo of the dextran-leptin conjugate was extended as compared to the circulation time of the unmodified rmetHu-leptin.

Similarly, the dextran-leptin conjugate injected s.c. persisted in the blood through 96 hours post-injection, whereas the unmodified leptin had a relatively rapid clearance of about 18-24 hours post-injection, as seen in Figure 5B.

### Example 4

This example demonstrates the increased solubility under physiologic conditions of the 6 kD dextran-leptin conjugates of the present invention as compared to unmodified rmetHu-leptin. Samples of the dextran-leptin conjugate and unmodified leptin were taken after protein purification. The samples were dialyzed overnight at 4°C into Dulbecco's PBS, pH 7. A dilute solution of each of the unmodified rmetHu-leptin and dextran-rmetHu-leptin conjugate (approximately 1-2 mg/mL leptin protein) was concentrated using an Amicon Centriprep™ 10 (Beverly, MA) concentration system. Samples were centrifuged in repeated cycles of decreasing duration at 4°C, 3400 rpm until a volume of approximately 0.5 mL was obtained. Protein concentration was determined in triplicate by the Bradford assay, Anal. Biochem. 72:248-54 (1976), using BioRad reagents (Hercules, CA). While the unmodified rmetHu-leptin solution would begin to show the protein precipitating at about 2-3 mg/mL in PBS at neutral pH, the dextran-leptin conjugate was still soluble at 80 mg/mL. At even higher concentrations, the dextran-leptin conjugate solution became difficult to handle. As well understood to one of ordinary skill in the art, parameters such as buffering system, temperature, surfactants and ionic strength may be adjusted to optimize solubility conditions.

### Example 5

This example demonstrates the minimal injection site reactions of the 6 kD dextran-leptin conjugates of the present invention. Normal female C57BL/6 mice(three mice per group) were injected once a day for seven days at a dose of either 25 mg/kg or 100 mg/kg (concentration of 5 mg/mL and 20 mg/mL, respectively) with the dextran-leptin conjugate (in PBS, pH 7.1) as prepared in Example 1 and unmodified rmetHu-leptin (in 10 mM sodium acetate buffer, 5% sorbitol, pH 4.0). Injection site samples were collected on the eighth day. The injection sites of the animals were analyzed for several histopathologic parameters: necrosis, inflammation (mononuclear versus suppurative), precipitated leptin, fibroplasia and giant cells. Based on such an evaluation, the dextran-leptin conjugate was very well tolerated at the injection site, even when injected at a 20 mg/mL concentration. There was no evidence of leptin precipitation and no necrosis of the tissue around the injection track. As compared to unmodified leptin, the injection site reaction was well tolerated at concentrations higher than the clinically acceptable concentration of unmodified leptin. The upper concentration limit for acceptable injection site reactions of the dextran-leptin conjugate has not yet been determined.

### Example 6

This example demonstrates the lack of an immunogenic response in primates to the 6 kD dextran-leptin conjugates of the present invention. Chimpanzees were treated for 4 weeks with a three times per week s.c. injection of 0.1 mg/kg 6 kD dextran-leptin conjugate as prepared in Example 1, and chimp sera were analyzed by ELISA. Little to no IgG or IgM response to the dextran-leptin conjugate was observed. A high background antibody level was observed and, thus, inhibition studies were performed to determine whether these antibodies were reactive against dextran (6 kD or 70 kD), the 6 kD dextran-leptin conjugate or the leptin protein itself. Inhibition studies with pre-bleed samples and end of study (Day 29) samples showed no significant differences in inhibition, indicating that the antibody reactivity was directed toward pre-formed antibodies. In particular, the 70 kD dextran showed full inhibition, while the 6 kD dextran showed partial inhibition. Furthermore, the dextran-leptin conjugate also showed full inhibition, while the unmodified leptin protein alone showed no inhibition. These results indicated that the antibody activity was directed against the dextran. Most likely, epitopes of the branched dextran (70 kD) were targeted. The results clearly showed that no antibody activity was directed to the leptin protein itself.

### Example 7

This example demonstrates the lack of kidney vacuole formation to the 6 kD dextran-leptin conjugates of the present invention as compared to prior art water soluble polymer conjugates such as mono-polyethylene glycol-leptin conjugates. Briefly, mice (three per group) were injected once a day for seven days at a dose of either 1 mg/kg or 10 mg/kg with the 6 kD dextran-leptin conjugate as prepared in Example 1 and unmodified rmetHu-leptin. Samples were collected on the eighth day. Tubular vacuole formation was scored. There was no induction of kidney vacuole formation with the dextran-leptin conjugate at either dose. The histopathology findings were comparable to those seen for unmodified leptin. In contrast, monopegylated leptin at 1 mg/kg and 10 mg/kg doses induced mild and marked vacuole formation, respectively, under the same conditions.

### Example 8

This example shows the preparation and weight-loss characteristics of another dextran-leptin conjugate of the present invention. The dextran leptin conjugate was prepared in accordance with the methods of Example 1 with the exception that 17.5 kD dextran was used in place of 6 kD dextran. The 17.5 kD dextran was purchased from Fluka Chemical Corp.

The 17.5 kD dextran-leptin conjugate was tested for efficacy in inducing weight loss in normal mice. Normal female C57BL/6 mice, age 8-10 weeks and weighing approximately 20 grams, were injected daily for seven days with either the subject 17.5 kD dextran-leptin conjugate, unmodified rmetHu-leptin or placebo (phosphate buffered saline, "PBS"). A 10 mg/kg dose was tested for both the 17.5 kD dextran-leptin conjugate and unmodified leptin. Weights were monitored throughout the study.

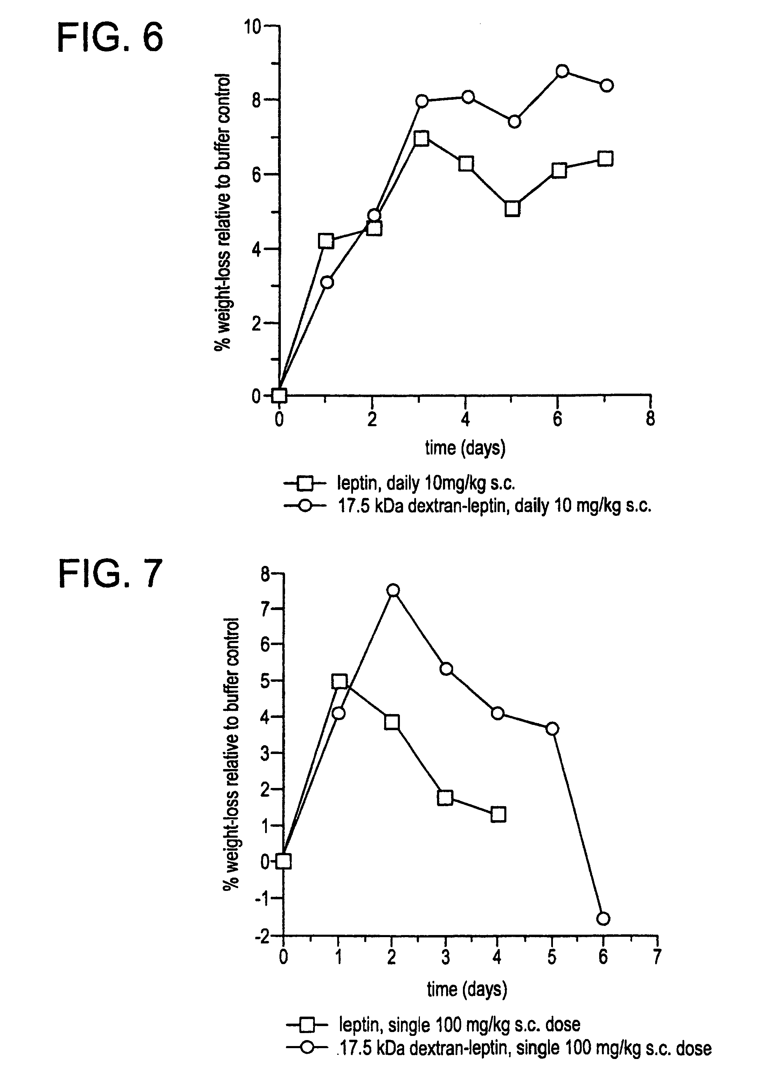

The weight change during the course of the study for the 17.5 kD dextran-leptin conjugate-treated group was compared against the unmodified leptin-treated group. As seen in Figure 6, the efficacy of the dextran-leptin conjugate was greater than that for the unmodified leptin at the 10 mg/kg dose.

The sustained weight-loss effect of the 17.5 kD dextran-rmetHu-leptin conjugate was also tested. Mice were injected s.c. on day 0 only at a dose of 100 mg/kg with either 17.5 kD dextran-rmetHu-leptin conjugate or unmodified rmetHu-leptin. As seen in Figure 7, the weight loss effect of the dextran-leptin conjugate lasted for 5-6 days with a one and one-half-fold increase in peak weight loss, whereas the weight loss effect of the unmodified leptin lasted only 3-4 days.

Thus, this example demonstrates the improved efficacy of the 17.5 kD dextran-leptin conjugates of the present invention over unmodified rmetHu-leptin for weight loss.

### Example 9

This example demonstrates the lack of kidney vacuolization formation to the 17.5 kD dextran-leptin conjugates of the present invention as compared to prior art water soluble polymer conjugates such as mono-polyethylene glycol-leptin conjugates. Mice were injected at a dose of either 1 mg/kg or 10 mg/kg with the 17.5 kD dextran-leptin conjugate or unmodified rmetHu-leptin in accordance with the procedures of Example 7. There was no induction of kidney vacuole formation with the 17.5 kD dextran-leptin conjugate at either dose. The histopathology findings were comparable to those seen for unmodified leptin.

### Example 10

This example envisions the lack of response of the dextran-leptin conjugates of the present invention with human antibodies from individuals susceptible to anaphylactic reactions with dextran. Experiments are performed to show that the dextran-leptin conjugates of the present invention are non-anaphylactic to individuals with high titers of circulating, pre-formed dextran-reactive antibodies.

Sera is collected from patients with high titers of circulating, pre-formed DRA who had experienced anaphylactic reactions (ARs) of varying severity. Sera from normal individuals is also collected as controls.

In vitro Testing: In vitro testing for DRA reactivity includes known methods such as passive hemagglutination. See, e.g., Hedin et al., Int. Arch. Allergy Appl. Immunol. 52:145-49 (1976), herein incorporated by reference. It has been shown that the titer of DRA is positively related to the degree of severity of ARs. Richter et al., supra, at 134-35.

For example, sera of dextran reactors are tested in a passive hemagglutination assay to determine whether DRA reactive sera react with the dextran-leptin conjugates of the present invention. Briefly, erythrocytes, to which dextran-leptin conjugates of the present invention have been absorbed onto their surface, as well as erythrocytes absorbed with unmodified leptin and low molecular weight dextran, separately, as negative controls and 70 kD dextran as a positive control, are incubated with DRA sera and then the erythrocytes are observed for signs of agglutination. No agglutination of the erythrocytes takes place because the DRA sera has reduced affinity for the low molecular weight dextrans used in the practice of this invention and/or the low molecular weight dextrans do not cause antibody aggregation and the cascade effects.

In vivo Testing: Alternatively, animal studies are conducted to determine DRA reactivity with the low molecular weight dextran-leptin conjugates of the present invention. The animals are first sensitized using a dextran cross-reactive antisera and/or antisera to high molecular weight dextran (e.g., 70 kD dextran) at a dose sufficient to elicit anaphylaxis. Next, the animals are challenged with a dextran-leptin conjugate of the present invention. The animals are observed for any signs of anaphylactic reactions such as skin manifestations, a rapid fall in blood pressure, respiratory distress, cardiac arrest, etc. No signs of anaphylactic reactions are observed as the pre-formed DRAs are not reactive with the low molecular weight dextrans used in the practice of this invention.

## Claims

1. A dextran-leptin conjugate comprising at least one low molecular weight dextran moiety attached to at least one leptin moiety wherein said dextran moiety has a molecular weight from 1 kD to 20 kD.

2. The dextran-leptin conjugate of claim 1 wherein said dextran moiety has a molecular weight from 1 kD to 10 kD.

3. The dextran-leptin conjugate of claim 2 wherein said dextran moiety has a molecular weight from 1 kD to 7 kD.

4. The dextran-leptin conjugate of claim 1 wherein said dextran moiety has a molecular weight of about 6 kD.

5. The dextran-leptin conjugate of claim 1 wherein one dextran moiety is attached to one or more leptin moieties.

6. The dextran-leptin conjugate of claim 1 wherein at least two dextran moieties are attached to one or more leptin moieties.

7. The dextran-leptin conjugate of claim 1 wherein multiple dextran moieties are attached to one leptin moiety.

8. The dextran-leptin conjugate of claim 1 wherein said leptin moiety is selected from the group consisting of (according to the amino acid sequence of SEQ ID NO: 1):
(a) the amino acid sequence of SEQ ID NO: 1, optionally lacking a glutaminyl residue at position 28, and further optionally having a methionyl residue at the N-terminus;
(b) an amino acid sequence of subpart (a) having a different amino acid substituted in one or more of the following positions: 4, 8, 32, 33, 35, 48, 50, 53, 60, 64, 66, 67, 68, 71, 74, 77, 78, 89, 97, 100, 102, 105, 106, 107, 108, 111, 112, 118, 136, 138, 142 and 145;
(c) an amino acid sequence of subpart (b) wherein the amino acids at positions 100 and 138 are substituted with Gln;
(d) a truncated leptin protein analog selected from among:
(i) amino acids 98-146
(ii) amino acids 1-99 and 112-146
(iii) amino acids 1-99 and 112-146 having one or more of amino acids 100-111 sequentially placed between amino acids 99 and 112; and,
(iv) the truncated leptin analog of subpart (i) having one or more of amino acids 100, 102, 105, 106, 107, 108, 111, 112, 118, 136, 138, 142 and 145 substituted with another amino acid;
(v) the truncated leptin analog of subpart (iii) having one or more of amino acids 4, 8, 32, 33, 35, 48, 50, 53, 60, 64, 66, 67, 68, 71, 74, 77, 78, 89, 97, 112, 118, 136, 138, 142 and 145 replaced with another amino acid;
(vi) the truncated leptin analog of subpart (iv) having one or more of amino acids 4, 8, 32, 33, 35, 48, 50, 53, 60, 64, 66, 67, 68, 71, 74, 77, 78, 89, 97, 100, 102, 105, 106, 107, 108, 111, 112, 118, 136, 138, 142 and 145 replaced with another amino acid; and
(vii) the truncated leptin analog of any of subparts (i)-(vi) having an N-terminal methionyl residue;
(e) a leptin protein of any of subparts (a)-(d) having one or more conserved amino acid substitutions.

9. A dextran-leptin conjugate mixture comprising the following in any combination:
(a) dextran-leptin conjugate wherein one dextran moiety is attached to one leptin moiety;
(b) dextran-leptin conjugate wherein one dextran moiety is attached to two or more leptin moieties;
(C) dextran-leptin conjugate wherein at least two dextran moieties are attached to one leptin moiety; and
(d) dextran-leptin conjugate wherein at least two dextran moieties are attached to at least two leptin moieties.

10. The dextran-leptin conjugate mixture of claim 9 comprising the following in any combination:
(a) dextran-leptin conjugate wherein one dextran moiety is attached to one leptin moiety;
(b) dextran-leptin conjugate wherein one dextran moiety is attached to two leptin moieties; and
(c) dextran-leptin conjugate wherein two dextran moieties are attached to two leptin moieties, said pair of dextran-leptin conjugates being attached to each other.

11. The dextran-leptin conjugate mixture of claim 10 consisting predominantly of:
(a) dextran-leptin conjugate wherein one dextran moiety is attached to two leptin moieties; and
(b) dextran-leptin conjugate wherein two dextran moieties are attached to two leptin moieties, said pair of dextran-leptin conjugates being attached to each other.

12. A dextran-leptin conjugate produced by the method comprising:
(a) activating a dextran moiety having a molecular weight from 1 kD to 20 kD;
(b) attaching the activated dextran moiety to a leptin moiety under reducing conditions to form an amine bond, at a pH sufficiently acidic so that the amino-terminal amine is not yet protonated while the amine groups at other positions on the leptin protein are protonated;
(c) obtaining the dextran-leptin conjugate; and
(d) optionally, purifying the dextran-leptin conjugate.

13. A pharmaceutical composition comprising a dextran-leptin conjugate according to any of claims 1 to 8 in a pharmaceutically acceptable carrier.

14. A pharmaceutical composition comprising a dextran-leptin conjugate mixture according to any of claims 9 to 11 in a pharmaceutically acceptable carrier.

15. Use of a composition for the manufacture of a medicament for the treatment of an individual for a condition selected from among: obesity, diabetes and hyperlipidemia, said composition comprising:
an effective amount of a dextran-leptin conjugate according to any of claims 1 to 8.

16. Use of a composition for the manufacture of a medicament for the treatment of an individual for a condition selected from among: obesity, diabetes and hyperlipidemia, said composition comprising:
an effective amount of a dextran-leptin conjugate mixture according to any of claims 9 to 11.

17. A dextran-leptin conjugate comprising at least one dextran moiety having a molecular weight from 1 kD to 20 kD attached at the N-terminus to at least one leptin moiety having the amino acid sequence of SEQ ID NO: 1, optionally lacking a glutaminyl residue at position 28, and further optionally having a methionyl residue at the N-terminus.

18. The dextran-leptin conjugate of claim 17 wherein said dextran moiety has a molecular weight of about 6 kD.

19. A dextran-leptin conjugate mixture comprising the following in any combination:
(a) dextran-leptin conjugate wherein one dextran moiety is attached to one leptin moiety;
(b) dextran-leptin conjugate wherein one dextran moiety is attached to two leptin moieties; and
(c) dextran-leptin conjugate wherein two dextran moieties are attached to two leptin moieties, said pair of dextran-leptin conjugates being attached to each other;
wherein said dextran moiety of said dextran-leptin conjugate has a molecular weight from 1 kD to 20 kD and is attached at the N-terminus to said leptin moiety having the amino acid sequence of SEQ ID NO: 1, optionally lacking a glutaminyl residue at position 28, and further optionally having a methionyl residue at the N-terminus.

20. The dextran-leptin conjugate mixture of claim 19 wherein said dextran moiety has a molecular weight of about 6 kD.

21. A pharmaceutical composition comprising a dextran-leptin conjugate according to any of claims 17 to 18 in a pharmaceutically acceptable carrier.

22. A dextran-leptin conjugate produced by the method comprising:
(a) activating a dextran moiety having a molecular weight from 1 kD to 20 kD;
(b) attaching the activated dextran moiety to a leptin moiety under reducing conditions to form an amine bond, at a pH sufficiently acidic so that the amino-terminal amine is not yet protonated while the amine groups at other positions on the leptin protein are protonated;
(c) obtaining the dextran-leptin conjugate; and
(d) optionally, purifying the dextran-leptin conjugate;
wherein said dextran moiety is attached at the N-terminus to said leptin moiety having the amino acid sequence of SEQ ID NO: 1, optionally lacking a glutaminyl residue at position 28, and further optionally having a methionyl residue at the N-terminus.

23. Use of a composition for the manufacture of a medicament for the treatment of an individual for a condition selected from among: obesity, diabetes and hyperlipidemia, said composition comprising:
an effective amount of a dextran-leptin conjugate according to any of claims 17 to 18.

24. A dextran-leptin conjugate comprising at least one dextran moiety having a molecular weight from 1 kD to 20 kD attached at the N-terminus to at least one leptin moiety having the amino acid sequence of SEQ ID NO: 1 wherein the amino acids at positions 100 and 138 are substituted with glutamine, optionally lacking a glutaminyl residue at position 28, and further optionally having a methionyl residue at the N-terminus.

25. The dextran-leptin conjugate of claim 24 wherein said dextran moiety has a molecular weight of about 6 kD.

26. A dextran-leptin conjugate mixture comprising the following in any combination:
(a) dextran-leptin conjugate wherein one dextran moiety is attached to one leptin moiety;
(b) dextran-leptin conjugate wherein one dextran moiety is attached to two leptin moieties; and
(c) dextran-leptin conjugate wherein two dextran moieties are attached to two leptin moieties, said pair of dextran-leptin conjugates being attached to each other;
wherein said dextran moiety of said dextran-leptin conjugate has a molecular weight from 1 kD to 20 kD and is attached at the N-terminus to said leptin moiety having the amino acid sequence of SEQ ID NO: 1 wherein the amino acids at positions 100 and 138 are substituted with glutamine, optionally lacking a glutaminyl residue at position 28, and further optionally having a methionyl residue at the N-terminus.

27. The dextran-leptin conjugate mixture of claim 26 wherein said dextran moiety has a molecular weight of about 6 kD.

28. A pharmaceutical composition comprising a dextran-leptin conjugate according to any of claims 24 to 25 in a pharmaceutically acceptable carrier.

29. A dextran-leptin conjugate produced by the method comprising:
(a) activating a dextran moiety having a molecular weight from 1 kD to 20 kD;
(b) attaching the activated dextran moiety to a leptin moiety under reducing conditions to form an amine bond, at a pH sufficiently acidic so that the amino-terminal amine is not yet protonated while the amine groups at other positions on the leptin protein are protonated;
(c) obtaining the dextran-leptin conjugate; and
(d) optionally, purifying the dextran-leptin conjugate;
wherein said dextran moiety is attached at the N-terminus to said leptin moiety having the amino acid sequence of SEQ ID NO: 1
wherein the amino acids at positions 100 and 138 are substituted with glutamine, optionally lacking a glutaminyl residue at position 28, and further optionally having a methionyl residue at the N-terminus.

30. Use of a composition for the manufacture of a medicament for the treatment of an individual for a condition selected from among: obesity, diabetes and hyperlipidemia, said composition comprising:
an effective amount of dextran-leptin conjugate according to any of claims 24 to 25.

## Patentansprüche

1. Dextran-Leptin-Konjugat, welches wenigstens eine niedermolekulargewichtige Dextran-Einheit umfaßt, die an wenigstens eine Leptin-Einheit angefügt ist, wobei die Dextran-Einheit ein Molekulargewicht von 1 kD bis 20 kD aufweist.

2. Dextran-Leptin-Konjugat nach Anspruch 1, **dadurch gekennzeichnet, daß** die Dextran-Einheit ein Molekulargewicht von 1 kD bis 10 kD aufweist.

3. Dextran-Leptin-Konjugat nach Anspruch 2, **dadurch gekennzeichnet, daß** die Dextran-Einheit ein Molekulargewicht von 1 kD bis 7 kD aufweist.

4. Dextran-Leptin-Konjugat nach Anspruch 1, **dadurch gekennzeichnet, daß** die Dextran-Einheit ein Molekulargewicht von etwa 6 kD aufweist.

5. Dextran-Leptin-Konjugat nach Anspruch 1, **dadurch gekennzeichnet, daß** eine Dextran-Einheit an einer oder mehreren Leptin-Einheiten angefügt ist.

6. Dextran-Leptin-Konjugat nach Anspruch 1, **dadurch gekennzeichnet, daß** wenigstens zwei Dextran-Einheiten an einer oder mehreren Leptin-Einheiten angefügt sind.

7. Dextran-Leptin-Konjugat nach Anspruch 1, **dadurch gekennzeichnet, daß** mehrere Dextran-Einheiten an einer Leptin-Einheit angefügt sind.

8. Dextran-Leptin-Konjugat nach Anspruch 1, **dadurch gekennzeichnet, daß** die Leptin-Einheit ausgewählt ist aus der Gruppe bestehend aus (gemäß der Aminosäuresequenz SEQ ID NO: 1):
(a) der Aminosäuresequenz SEQ ID NO: 1, optional unter Fehlen eines Glutaminylrests an Position 28 und weiter optional mit einem Methionylrest an dem N-Ende;
(b) einer Aminosäuresequenz von Unterteil (a) mit einer unterschiedlichen Aminosäure, die an einer oder mehreren der folgenden Positionen substituiert ist: 4, 8, 32, 33, 35, 48, 50, 53, 60, 64, 66, 67, 68, 71, 74, 77, 78, 89, 97, 100, 102, 105, 106, 107, 108, 111, 112, 118, 136, 138, 142 und 145;
(c) einer Aminosäuresequenz von Unterteil (b), wobei die Aminosäuren an Positionen 100 und 138 mit Gln substituiert sind;
(d) einem abgestutzten Leptin-Proteinanalogon, das ausgewählt ist aus:
(i) Aminosäuren 98-146
(ii) Aminosäuren 1-99 und 112-146
(iii) Aminosäuren 1-99 und 112-146 mit einer oder mehreren Aminosäuren 100-111, die sequentiell zwischen Aminosäuren 99 und 112 angeordnet sind; und
(iv) dem abgestutzten Leptinanalogon von Unterteil (i) mit einer oder mehreren Aminosäuren 100, 102, 105, 106, 107, 108, 111, 112, 118, 136, 138, 142 und 145, die mit einer anderen Aminosäure substituiert ist bzw. sind;
(v) dem abgestutzten Leptinanalogon von Unterteil (iii) mit einer oder mehreren Aminosäuren 4, 8, 32, 33, 35, 48, 50, 53, 60, 64, 66, 67, 68, 71, 74, 77, 78, 89, 97, 112, 118, 136, 138, 142 und 145, die durch eine andere Aminosäure ersetzt ist bzw. sind;
(vi) dem abgestutzten Leptinanalogon von Unterteil von (iv) mit einer oder mehreren Aminosäuren 4, 8, 32, 33, 35, 48, 50, 53, 60, 64, 66, 67, 68, 71, 74, 77, 78, 89, 97, 100, 102, 105, 106, 107, 108, 111, 112, 118, 136, 138, 142 und 145, die durch eine andere Aminosäure ersetzt ist bzw. sind; und
(vii) dem abgestutzten Leptinanalogon von jedem der Unterteile (i) - (vi) mit einem N-terminalen Methionylrest;
(e) einem Leptin-Protein jedes der Unterteile (a) - (d) mit einer oder mehreren aufrechterhaltenen Aminosäuresubstitutionen.

9. Dextrin-Leptin-Konjugatmischung, welche die folgenden in irgendeiner Kombination umfaßt:
(a) Dextran-Leptin-Konjugat, wobei eine Dextran-Einheit an einer Leptin-Einheit angefügt ist;
(b) Dextran-Leptin-Konjugat, wobei eine Dextran-Einheit an zwei oder mehreren Leptin-Einheiten angefügt ist;
(c) Dextran-Leptin-Konjugat, wobei wenigstens zwei Dextran-Einheiten an einer Leptin-Einheit angefügt sind; und
(d) Dextran-Leptin-Konjugat, wobei wenigstens zwei Dextran-Einheiten an wenigstens zwei Leptin-Einheiten angefügt sind.

10. Dextran-Leptin-Konjugatmischung nach Anspruch 9, welche die folgenden in irgendeiner Kombination umfaßt:
(a) Dextran-Leptin-Konjugat, wobei eine Dextran-Einheit an einer Leptin-Einheit angefügt ist;
(b) Dextran-Leptin-Konjugat, wobei eine Dextran-Einheit an zwei Leptin-Einheiten angefügt ist; und
(c) Dextran-Leptin-Konjugat, wobei zwei Dextran-Einheiten an zwei Leptin-Einheiten angefügt sind, wobei das Paar von Dextran-Leptin-Konjugaten aneinander angefügt ist.

11. Dextran-Leptin-Konjugatmischung nach Anspruch 10, die vorherrschend besteht aus:
(a) Dextran-Leptin-Konjugat, wobei eine Dextran-Einheit an zwei Leptin-Einheiten angefügt ist; und
(b) Dextran-Leptin-Konjugat, wobei zwei Dextran-Einheiten an zwei Leptin-Einheiten angefügt sind, wobei das Paar von Dextran-Leptin-Konjugaten aneinander angefügt ist.

12. Dextran-Leptin-Konjugat, welches durch das Verfahren hergestellt wird, welches umfaßt:
(a) Aktivieren einer Dextran-Einheit mit einem Molekulargewicht von 1 kD bis 20 kD;
(b) Anfügen der aktivierten Dextran-Einheit an einer Leptin-Einheit unter reduzierenden Bedingungen, um eine Aminbindung zu bilden, bei einem pH-Wert, der ausreichend sauer ist, so daß das Amino-terminale Amin noch nicht protoniert wird, während die Amingruppen an anderen Positionen an dem Leptinprotein protoniert werden;
(c) Erhalten des Dextran-Leptin-Konjugats; und
(d) optional Reinigen des Dextran-Leptin-Konjugats.

13. Pharmazeutische Zusammensetzung, welche ein Dextran-Leptin-Konjugat nach einem der Ansprüche 1 bis 8 in einem pharmazeutisch annehmbaren Träger umfaßt.

14. Pharmazeutische Zusammensetzung, welche eine Dextran-Leptin-Konjugatmischung nach einem der Ansprüche 9 bis 11 in einem pharmazeutisch annehmbaren Träger umfaßt.

15. Verwendung einer Zusammensetzung für die Herstellung eines Medikaments für die Behandlung eines Individuums für einen Zustand, der ausgewählt ist unter: Fettleibigkeit, Diabetes und Hyperlipidämie, wobei die Zusammensetzung umfaßt:
eine wirksame Menge eines Dextran-Leptin-Konjugats nach einem der Ansprüche 1 bis 8.

16. Verwendung einer Zusammensetzung für die Herstellung eines Medikaments für die Behandlung eines Individuums für einen Zustand, der ausgewählt ist unter: Fettleibigkeit, Diabetes und Hyperlipidämie, wobei die Zusammensetzung umfaßt:
eine wirksame Menge einer Dextran-Leptin-Konjugatmischung nach einem der Ansprüche 9 bis 11.

17. Dextran-Leptin-Konjugat, welches wenigstens eine Dextran-Einheit mit einem Molekulargewicht von 1 kD bis 20 kD umfaßt, angefügt an dem N-Ende an wenigstens einer Leptin-Einheit mit der Aminosäuresequenz SEQ ID NO: 1, optional unter Fehlen eines Glutaminylrests an Position 28 und ferner optional mit einem Methionylrest an dem N-Ende.

18. Dextran-Leptin-Konjugat nach Anspruch 17, **dadurch gekennzeichnet, daß** die Dextran-Einheit ein Molekulargewicht von etwa 6 kD aufweist.

19. Dextran-Leptin-Konjugatmischung, welche die folgenden in irgendeiner Kombination umfaßt:
(a) Dextran-Leptin-Konjugat, wobei eine Dextran-Einheit an einer Leptin-Einheit angefügt ist;
(b) Dextran-Leptin-Konjugat, wobei eine Dextran-Einheit an zwei Leptin-Einheiten angefügt ist; und
(c) Dextran-Leptin-Konjugat, wobei zwei Dextran-Einheiten an zwei Leptin-Einheiten angefügt sind, wobei das Paar von Dextran-Leptin-Konjugaten aneinander angefügt ist;
wobei die Dextran-Einheit des Dextran-Leptin-Konjugats ein Molekulargewicht von 1 kD bis 20 kD aufweist und an dem N-Ende an der Leptin-Einheit mit der Aminosäuresequenz SEQ ID NO: 1 angefügt ist, optional unter Fehlen eines Glutaminylrests an Position 28 und ferner optional mit einem Methionylrest an dem N-Ende.

20. Dextran-Leptin-Konjugatmischung nach Anspruch 19, **dadurch gekennzeichnet, daß** die Dextran-Einheit ein Molekulargewicht von etwa 6 kD aufweist.

21. Pharmazeutische Zusammensetzung, welche ein Dextran-Leptin-Konjugat nach einem der Ansprüche 17 bis 18 in einem pharmazeutisch annehmbaren Träger umfaßt.

22. Dextran-Leptin-Konjugat, welches durch das Verfahren hergestellt wird, welches umfaßt:
(a) Aktivieren einer Dextran-Einheit mit einem Molekulargewicht von 1 kD bis 20 kD;
(b) Anfügen der aktivierten Dextran-Einheit an einer Leptin-Einheit unter reduzierenden Bedingungen, um eine Aminbindung zu bilden, bei einem pH-Wert, der ausreichend sauer ist, so daß das Amino-terminale Amin noch nicht protoniert wird, während die Amingruppen an anderen Positionen an dem Leptin-Protein protoniert werden;
(c) Erhalten des Dextran-Leptin-Konjugats; und
(d) optional Reinigen des Dextran-Leptin-Konjugats;
wobei die Dextran-Einheit an dem N-Ende an der Leptin-Einheit mit der Aminosäuresequenz SEQ ID NO: 1 angefügt wird, optional unter Fehlen eines Glutaminylrests an Position 28 und ferner optional mit einem Methionylrest an dem N-Ende.

23. Verwendung einer Zusammensetzung für die Herstellung eines Medikaments für die Behandlung eines Individuums für einen Zustand, der ausgewählt ist unter: Fettleibigkeit, Diabetes und Hyperlipidämie, wobei die Zusammensetzung umfaßt:
eine wirksame Menge eines Dextran-Leptin-Konjugats nach einem der Ansprüche 17 bis 18.

24. Dextran-Leptin-Konjugat, welches wenigstens eine Dextran-Einheit mit einem Molekulargewicht von 1 kD bis 20 kD, angefügt an dem N-Ende an wenigstens einer Leptin-Einheit mit der Aminosäuresequenz SEQ ID NO: 1 umfaßt, wobei die Aminosäuren an Positionen 100 und 138 mit Glutamin substituiert sind, optional unter Fehlen eines Glutaminylrests an Position 28 und ferner optional mit einem Methionylrest an dem N-Ende.

25. Dextran-Leptin-Konjugat nach Anspruch 24, **dadurch gekennzeichnet, daß** die Dextran-Einheit ein Molekulargewicht von etwa 6 kD aufweist.

26. Dextran-Leptin-Konjugatmischung, welche die folgenden in irgendeiner Kombination umfaßt:
(a) Dextran-Leptin-Konjugat, wobei eine Dextran-Einheit an einer Leptin-Einheit angefügt ist;
(b) Dextran-Leptin-Konjugat, wobei eine Dextran-Einheit an zwei Leptin-Einheiten angefügt ist; und
(c) Dextran-Leptin-Konjugat, wobei zwei Dextran-Einheiten an zwei Leptin-Einheiten angefügt sind, wobei das Paar von Dextran-Leptin-Konjugaten aneinander angefügt ist;
wobei die Dextran-Einheit des Dextran-Leptin-Konjugats ein Molekulargewicht von 1 kD bis 20 kD aufweist und an dem N-Ende an der Leptin-Einheit mit der Aminosäuresequenz SEQ ID NO: 1 angefügt ist, wobei die Aminosäuren an Positionen 100 und 138 mit Glutamin substituiert sind, optional unter Fehlen eines Glutaminylrests an Position 28 und ferner optional mit einem Methionylrest an dem N-Ende.

27. Dextran-Leptin-Konjugatmischung nach Anspruch 26, **dadurch gekennzeichnet, daß** die Dextran-Einheit ein Molekulargewicht von etwa 6 kD aufweist.

28. Pharmazeutische Zusammensetzung, welche ein Dextran-Leptin-Konjugat nach einem der Ansprüche 24 bis 25 in einem pharmazeutisch annehmbaren Träger umfaßt.

29. Dextran-Leptin-Konjugat, welches durch das Verfahren hergestellt wird, welches umfaßt:
(a) Aktivieren einer Dextran-Einheit mit einem Molekulargewicht von 1 kD bis 20 kD;
(b) Anfügen der aktivierten Dextran-Einheit an einer Leptin-Einheit unter reduzierenden Bedingungen, um eine Aminbindung zu bilden, bei einem pH-Wert, der ausreichend sauer ist, so daß das Amino-terminale Amin noch nicht protoniert wird, während die Amingruppen an anderen Positionen an dem Leptin-Protein protoniert werden;
(c) Erhalten des Dextran-Leptin-Konjugats; und
(d) optional Reinigen des Dextran-Leptin-Konjugats;
wobei die Dextran-Einheit an dem N-Ende an der Leptin-Einheit mit der Aminosäuresequenz SEQ ID NO: 1 angefügt wird, wobei die Aminosäuren an Positionen 100 und 138 mit Glutamin substituiert sind, optional unter Fehlen eines Glutaminylrests an Position 28 und ferner optional mit einem Methionylrest an dem N-Ende.

30. Verwendung einer Zusammensetzung für die Herstellung eines Medikaments für die Behandlung eines Individuums für einen Zustand, der ausgewählt wird unter: Fettleibigkeit, Diabetes und Hyperlipidämie, wobei die Zusammensetzung umfaßt:
eine wirksame Menge an Dextran-Leptin-Konjugat nach einem der Ansprüche 24 bis 25.

## Revendications

1. Conjugué dextrane-leptine comprenant au moins une fraction de dextrane à faible masse moléculaire fixée à au moins une fraction de leptine, dans lequel ladite fraction de dextrane a une masse moléculaire de 1 kD à 20 kD.

2. Conjugué dextrane-leptine de la revendication 1, dans lequel ladite fraction de dextrane a une masse moléculaire de 1 kD à 10 kD.

3. Conjugué dextrane-leptine de la revendication 2, dans lequel ladite fraction de dextrane a une masse moléculaire de 1 kD à 7 kD.

4. Conjugué dextrane-leptine de la revendication 1, dans lequel ladite fraction de dextrane a une masse moléculaire d'environ 6 kD.

5. Conjugué dextrane-leptine de la revendication 1, dans lequel une fraction de dextrane est fixée à une ou plusieurs fractions de leptine.

6. Conjugué dextrane-leptine de la revendication 1, dans lequel au moins deux fractions de dextrane sont fixées à une ou plusieurs fractions de leptine.

7. Conjugué dextrane-leptine de la revendication 1, dans lequel de multiples fractions de dextrane sont fixées à une fraction de leptine.

8. Conjugué dextrane-leptine de la revendication 1, dans lequel ladite fraction de leptine est choisie à partir du groupe constitué de (selon la séquence d'acides aminés de la SEQ ID N°1) :
(a) la séquence d'acides aminés de la SEQ ID N°1, éventuellement dépourvue d'un résidu de glutaminyl à la position 28, et ayant de plus éventuellement un résidu de méthionyl à la terminaison N ;
(b) une séquence d'acides aminés de sous-partie (a) ayant un acide aminé différent substitué dans une ou plusieurs des positions suivantes : 4, 8, 32, 33, 35, 48, 50, 53, 60, 64, 66, 67, 68, 71, 74, 77, 78, 89, 97, 100, 102, 105, 106, 107, 108, 111, 112, 118, 136, 138, 142 et 145 ;
(c) une séquence d'acides aminés de sous-partie (b) dans laquelle les acides aminés aux positions 100 et 138 sont substitués par Gln ;
(d) un analogue de protéine de leptine tronqué, choisi parmi ;
(i) les acides aminés 98-146
(ii) les acides aminés 1-99 et 112-146
(iii) les acides aminés 1-99 et 112-146 ayant un ou plusieurs des acides aminés 100-111 placés séquentiellement entre les acides aminés 99 et 112 ; et
(iv) l'analogue de leptine tronqué de sous-partie (i) ayant un ou plusieurs des acides aminés 100, 102, 105, 106, 107, 108, 111, 112, 118, 136, 138, 142 et 145 substitués par un autre acide aminé ;
(v) l'analogue de leptine tronqué de sous-partie (iii) ayant un ou plusieurs des acides aminés 4, 8, 32, 33, 35, 48, 50, 53, 60, 64, 66, 67, 68, 71, 74, 77, 78, 89, 97, 112, 118, 136, 138, 142 et 145 remplacés par un autre acide aminé ;
(vi) l'analogue de leptine tronqué de sous-partie (iv) ayant un ou plusieurs des acides aminés 4, 8, 32, 33, 35, 48, 50, 53, 60, 64, 66, 67, 68, 71, 74, 77, 78, 89, 97, 100, 102, 105, 106, 107, 108, 111, 112, 118, 136, 138, 142 et 145 remplacés par un autre acide aminé ; et
(vii) l'analogue de leptine tronqué d'une quelconque des sous-parties (i)-(vi) ayant un résidu de méthionyl N-terminal ;
(e) une protéine de leptine d'une quelconque des sous-parties (a)-(d) ayant une ou plusieurs des substitutions d'acides aminés conservées.

9. Mélange de conjugués dextrane-leptine comprenant la suite dans une quelconque combinaison :
(a) un conjugué dextrane-leptine dans lequel une fraction de dextrane est fixée à une fraction de leptine ;
(b) un conjugué dextrane-leptine dans lequel une fraction de dextrane est fixée à deux ou plusieurs fractions de leptine ;
(c) un conjugué dextrane-leptine dans lequel au moins deux fractions de dextrane sont fixées à une fraction de leptine ; et
(d) un conjugué dextrane-leptine dans lequel au moins deux fractions de dextrane sont fixées à au moins deux fractions de leptine.

10. Mélange de conjugués dextrane-leptine de la revendication 9 comprenant la suite dans une quelconque combinaison :
(a) un conjugué dextrene-leptine dans lequel une fraction de dextrane est fixée à une fraction de leptine ;
(b) un conjugué dextrane-leptine dans lequel une fraction de dextrane est fixée à deux fractions de leptine ; et
(c) un conjugué dextrane-leptine dans lequel deux fractions de dextrane sont fixées à deux fractions de leptine, ladite paire de conjugués dextrane-leptine étant fixée l'un à l'autre.

11. Mélange de conjugués dextrane-leptine de la revendication 10 composé de façon prédominante de :
(a) un conjugué dextrane-leptine dans lequel une fraction de dextrane est fixée à deux fractions de leptine ; et
(b) un conjugué dextrene-leptine dans lequel deux fractions de dextrane sont fixées à deux fractions de leptine, ladite paire de conjugués dextrane-leptine étant fixée l'un à l'autre.

12. Conjugué dextrane-leptine produit par le procédé consistant à :
(a) activer une fraction de dextrane ayant une masse moléculaire de 1 kD à 20 kD ;
(b) fixer la fraction de dextrane activée à une fraction de leptine sous des conditions réductrices pour former une liaison amine, à un pH suffisamment acide de sorte que l'amine amino-terminale n'est pas encore protonée tandis que les groupes amines aux autres positions sur la protéine de leptine sont protonés ;
(c) obtenir le conjugué dextrane-leptine ; et
(d) éventuellement, purifier le conjugué dextrane-leptine.

13. Composition pharmaceutique comprenant un conjugué dextrane-leptine selon l'une quelconque des revendications 1 à 8 dans un support pharmaceutiquement acceptable.

14. Composition pharmaceutique comprenant un mélange de conjugués dextrane-leptine selon une quelconque des revendications 9 à 11 dans un support pharmaceutiquement acceptable.

15. Utilisation d'une composition pour la fabrication d'un médicament pour le traitement d'un individu souffrant d'un état choisi parmi ; l'obésité, le diabète et l'hyperlipidémie, ladite composition comprenant : une quantité efficace d'un conjugué dextrane-leptine selon une quelconque des revendications 1 à 8.

16. Utilisation d'une composition pour la fabrication d'un médicament pour le traitement d'un individu souffrant d'un état choisi parmi ; l'obésité, le diabète et l'hyperlipidémie, ladite composition comprenant : une quantité efficace d'un mélange de conjugués dextrane-leptine selon une quelconque des revendications 9 à 11.

17. Conjugué dextrane-leptine comprenant au moins une fraction de dextrane ayant une masse moléculaire de 1 kD à 20 kD liée, à la terminaison N, à au moins une fraction de leptine ayant la séquence d'acides aminés de la SEQ ID N°1, éventuellement dépourvue d'un résidu de glutaminyl à la position 28, et ayant de plus éventuellement un résidu de méthionyl à la terminaison N.

18. Conjugué dextrane-leptine de la revendication 17, dans lequel ladite fraction de dextrane a une masse moléculaire d'environ 6 kD.

19. Mélange de conjugués dextrane-leptine comprenant la suite dans une quelconque combinaison :
(a) un conjugué dextrane-leptine dans lequel une fraction de dextrane est fixée à une fraction de leptine ;
(b) un conjugué dextrane-leptine dans lequel une fraction de dextrane est fixée à deux fractions de leptine ; et
(c) un conjugué dextrane-leptine dans lequel deux fractions de dextrane sont fixées à deux fractions de leptine, ladite paire de conjugués dextrane-leptine étant fixée l'un à l'autre ;
dans lequel ladite fraction de dextrane dudit conjugué dextrane-leptine a une masse moléculaire de 1 kD à 20 kD et est fixée, à la terminaison N, à ladite fraction de leptine ayant la séquence d'acides aminés de la SEQ ID N° 1, éventuellement dépourvue d'un résidu de glutaminyl à la position 28, et ayant de plus éventuellement un résidu de méthionyl à la terminaison N.

20. Mélange de conjugués dextrane-leptine de la revendication 19, dans lequel ladite fraction de dextrane a une masse moléculaire d'environ 6 kD.

21. Composition pharmaceutique comprenant un conjugué dextrane-leptine selon une quelconque des revendications 17 à 18 dans un support pharmaceutiquement acceptable.

22. Conjugué dextrane-leptine produit par le procédé consistant à :
(a) activer une fraction de dextrane ayant une masse moléculaire de 1 kD à 20 kD ;
(b) fixer la fraction de dextrane activée à une fraction de leptine sous des conditions réductrices pour former une liaison amine, à un pH suffisamment acide de sorte que l'amine amino-terminale n'est pas encore protonée tandis que les groupes amines aux autres positions sur la protéine de leptine sont protonés ;
(c) obtenir le conjugué dextrane-leptine ; et
(d) éventuellement, purifier le conjugué dextrane-leptine ;
dans lequel ladite fraction de dextrane est fixée, à la terminaison N, à ladite fraction de leptine ayant la séquence d'acides aminés de la SEQ ID N°1, éventuellement dépourvue d'un résidu de glutaminyl à la position 28, et ayant de plus éventuellement un résidu de méthionyl à la terminaison N.

23. Utilisation d'une composition pour la fabrication d'un médicament pour le traitement d'un individu souffrant d'un état choisi parmi : l'obésité, le diabète et l'hyperlipidémie, ladite composition comprenant :
une quantité efficace d'un conjugué dextrane-leptine selon une quelconque des revendications 17 à 18.

24. Conjugué dextrane-teptine comprenant au moins une fraction de dextrane ayant une masse moléculaire de 1 kD à 20 kD fixée, à la terminaison N, à au moins une fraction de leptine ayant la séquence d'acides aminés de la SEQ ID N°1 dans laquelle les acides aminés aux positions 100 et 138 sont substitués par la glutamine, éventuellement dépourvue d'un résidu de glutaminyl à la position 28, et ayant de plus éventuellement un résidu de méthionyl à la terminaison N.

25. Conjugué dextrane-leptine de la revendication 24, dans lequel ladite fraction de dextrane a une masse moléculaire d'environ 6 kD.

26. Mélange de conjugués dextrane-leptine comprenant ce qui suit dans une quelconque combinaison :
(a) un conjugué dextrane-leptine dans lequel une fraction de dextrane est fixée à une fraction de leptine ;
(b) un conjugué dextrane-leptine dans lequel une fraction de dextrane est fixée à deux fractions de leptine ; et
(c) un conjugué dextrane-leptine dans lequel deux fractions de dextrane sont fixées à deux fractions de leptine, ladite paire de conjugués dextrane-leptine étant fixée l'un à l'autre,
dans lequel ladite fraction de dextrane dudit conjugué dextrane-leptine a une masse moléculaire de 1 kD à 20 kD et est fixée, à la terminaison N, à ladite fraction de leptine ayant la séquence d'acides aminés de la SEQ ID N°1 dans laquelle les acides aminés aux positions 100 et 138 sont substitués par la glutamine, éventuellement dépourvue d'un résidu de glutaminyl à la position 28 et ayant de plus éventuellement un résidu de méthionyl à la terminaison N.

27. Mélange de conjugués dextrane-leptine de la revendication 26,
dans lequel ladite fraction de dextrane a une masse moléculaire d'environ 6 kD.

28. Composition pharmaceutique comprenant un conjugué dextrane-leptine selon une quelconque des revendications 24 à 25 dans un support pharmaceutiquement acceptable.

29. Conjugués dextrane-leptine produit par le procédé consistant à :
(a) activer une fraction de dextrane ayant une masse moléculaire de 1 kD à 20 kD ;
(b) fixer la fraction de dextrane activée à une fraction de leptine sous des conditions réductrices pour former une liaison amine, à un pH suffisamment acide de sorte que l'amine amino-terminale n'est pas encore protonée tandis que les groupes amines aux autres positions sur la protéine de leptine sont protonés ;
(c) obtenir le conjugué dextrane-leptine ; et
(d) éventuellement, purifier le conjugué dextrane-leptine ;
dans lequel ladite fraction de dextrane est fixée, à la terminaison N, à ladite fraction de leptine ayant la séquence d'acides aminés de la SEQ ID N°1, dans laquelle les acides aminés aux positions 100 et 138 sont substitués par la glutamine, éventuellement dépourvue d'un résidu de glutaminyl à la position 28, et ayant de plus éventuellement un résidu de méthionyl à la terminaison N.

30. Utilisation d'une composition pour la fabrication d'un médicament pour le traitement d'un individu souffrant d'un état choisi parmi ; l'obésité, le diabète et l'hyperlipidémie, ladite composition comprenant une quantité efficace d'un conjugué dextrane-leptine selon une quelconque des revendications 24 à 25.
